# EUROPÄISCHE PATENTSCHRIFT

(11) **EP 2 430 158 B1**
(45) Veröffentlichungstag und Bekanntmachung des Hinweises auf die Patenterteilung: **30.11.2016**
(21) Anmeldenummer: 10723505.3
(22) Anmeldetag: 11.05.2010
(51) Int. Cl.: C12N 15/10

(54) **NUKLEINSÄUREAUFREINIGUNGSVERFAHREN**
Nucleic acid purification method
Procédé de nettoyage d'acide nucléique

(30) Priorität: 12.05.2009 EP 09160078; 03.06.2009 EP 09007338
(43) Veröffentlichungstag der Anmeldung: 21.03.2012
(73) Patentinhaber: Qiagen GmbH, 40724 Hilden (DE)
(72) Erfinder: PETZEL, Jan, 40724 Hilden (DE); WEDLER, Holger, 40724 Hilden (DE); FABIS, Roland, 51375 Leverkusen (DE)
(74) Vertreter: Roth, Carla
(86) Internationale Anmeldenummer: PCT/EP2010/002875
(87) Internationale Veröffentlichungsnummer: WO 2010/130402

(56) Entgegenhaltungen:
- WO-A1-2006/128776
- WO-A1-2010/072821
- WO-A2-00/69872
- WO-A2-02/48164
- WO-A2-99/29703
- US-A- 5 718 892
- US-A1- 2005 130 196
- Johnson et al.: "'Factory-Proof' long PCR enrichment and amplicon equalization for next generation resequencing applications" 2008, XP002570305 Gefunden im Internet: URL:https://www.invitrogen.com/etc/mediali b/en/filelibrary/Nucleic-Acid-Amplificatio n-Expression-Profiling/PDFs.Par.12017.File .dat/O-076831_SequalPrep%20Application%20N ote%201_0508.pdf [gefunden am 2010-02-24]
- ANONYMOUS: 'SequalPrep (TM) Normalization Plate (96) Kit' INTERNET ARTICLE, [Online] 01 Januar 2008, Seiten 1 - 4, XP055174309 Gefunden im Internet: <URL:http://tools.lifetechnologies.com/cont ent/sfs/manuals/sequalprep_platekit_man.pdf > [gefunden am 2015-03-05]

## Beschreibung

Die vorliegende Erfindung betrifft ein Verfahren zur Aufreinigung einer definierten Menge von Nukleinsäuren aus einer nukleinsäurehaltigen Probe. Darüber hinaus werden geeignete Kits und nukleinsäurebindende Phasen zur Verfügung gestellt, die die Isolierung einer definierten Menge an Nukleinsäuren aus einer Probe erlauben und daher zur Normalisierung der Nukleinsäurekonzentration im Eluat geeignet sind.

Verschiedene Verfahren zur Aufreinigung und Isolierung von Nukleinsäuren sind im Stand der Technik bekannt. Diese beinhalten den Einsatz von Phenol-Chloroform, Aussalzverfahren, den Einsatz von Ionenaustauschern sowie Silikapartikel. Ein bekanntes Verfahren zur Nukleinsäureaufreinigung ist das sog. "Charge-Switch Verfahren". Danach wird eine nukleinsäurebindende Phase bei einem ersten pH-Wert mit einer nukleinsäurehaltigen Probe in Kontakt gebracht, bei der die nukleinsäurebindende Phase eine positive Ladung aufweist. Dies begünstigt die Bindung der negativ geladenen Nukleinsäuren an die Phase. Zur Freisetzung/Elution der Nukleinsäuren wird gemäß dem Charge-Switch Prinzip ein zweiter pH-Wert eingestellt, der höher als der pKs-Wert der nukleinsäurebindende Phase ist, um die positive Ladung zu invertieren, bzw. zu neutralisieren. Dies fördert die Ablösung der gebundenen Nukleinsäuren von der nukleinsäurebindenden Phase.

Für viele Analysetechniken und biologischen Verfahren muss eine bestimmte, d. h. definierte Menge an Nukleinsäure eingesetzt werden. Die Menge der isolierten oder amplifizierten Nukleinsäure aus unterschiedlichen Ausgangsmaterialien (Proben) hängt von einer Reihe schwer zu kontrollierender Faktoren ab, so dass die Ausbeuten je nach Methode und Material stark schwanken können. Um nachfolgende Experimente mit den isolierten Nukleinsäuren unter vergleichbaren Bedingungen durchführen zu können, ist in der Regel eine Quantifizierung der gewonnenen Nukleinsäure und anschließend ein Einstellen der Konzentrationen bzw. Nukleinsäuremenge auf einen bestimmten Wert und damit eine Normalisierung erforderlich. Für viele standardisierte Prozesse und insbesondere die Automatisierung von Prozessen ist eine solche Normalisierung oftmals zwingend notwendig, um reproduzierbare Ergebnisse zu erhalten.

Aus diesem Grunde gibt es eine Vielzahl von Quantifizierungsmethoden, um Nukleinsäuren in einer aufgereinigten Probe zu quantifizieren. Ein Beispiel für eine übliche Quantifizierungsmethode ist die spektrophotometrische Bestimmung der DNA Menge in einer Probe. Im Anschluss an die Bestimmung der Konzentration der Nukleinsäure in der Probe kann die Konzentration dann einheitlich angepasst werden. Andere bekannte Nukleinsäurequantifizierungsmethoden basieren auf interkalierenden Farbstoffen wie bspw. Ethidiumbromid, SYBR Green oder Picogreen. Durch Vergleich einer Standardkurve mit den gemessenen Werten kann die Konzentration bestimmt werden.

Neben den beschriebenen spektrometrischen oder auf Fluoreszenz basierenden Quantifizierungsmethoden sind im Stand der Technik auch verschiedene Aufreinigungsverfahren bekannt, die eine Präparation von einheitlichen Konzentrationen an Nukleinsäuren aus unterschiedlichen Ausgangsmaterialien erlauben sollen. Beispiele sind das MagneSil-Kit von Promega oder das SequalPrep-Kit von Invitrogen.

Das MagneSil-Kit von Promega wird bspw. verwendet, um genomische DNA aus Vollblut zu isolieren. Das Kit basiert auf der Bindung von DNA an Silika beschichtete paramagnetische Partikel unter chaotropen Salzbedingungen. Als Ergebnis erhält man üblicher Weise 1 µg (+/- 50 %) gereinigte genomische DNA nach Elution mit einem Niedrigsalzpuffer oder Wasser. Aufgrund der recht großen Schwankungen von bis zu 50 % ist diese Methode jedoch verbesserungsbedürftig.

Das SequalPrep-Kit von Invitrogen basiert auf der Bindung von DNA an Oberflächen, die mit einem Ionenaustauscher beschichtet sind. Die DNA bindet bei einem sauren pH-Wert und die Elution erfolgt mit einem stark alkalischen Puffer, dessen pH-Wert über dem pks-Wert des Ionenaustauschers liegt. Dieses Kit basiert auf der sogenannten Charge-Switch Technologie (siehe oben). Anwendung findet das Kit bei der Aufreinigung und Normalisierung von long range (LR) PCRs und es ist auch für die Template Vorbereitung für Sequenzierungen geeignet. Der Hersteller gibt die Ausbeuten von ca. 25 ng mit einer zwei bis dreifachen Schwankung bei mindestens 250 ng Ausgangs-DNA an. Das bedeutet, dass auch hier die Schwankungen recht hoch sein können, so dass auch hier Optimierungsbedarf besteht.

Die bekannten Aufreinigungsverfahren weisen daher in der Praxis immer noch starke Schwankungen in den Ausbeuten auf. Schwierigkeiten treten insbesondere dann auf, wenn unterschiedliche Mengen an Ausgangsmaterial oder unterschiedliche Ausgangsmaterialien zum Einsatz kommen.

Neben dem Erhalt einer aufgereinigten Nukleinsäure ist es daher wünschenswert, bei der Isolierung auch eine definierte Menge an Nukleinsäuren aufzureinigen und damit für ein Probenmaterial bereits ein Eluat zu erhalten, das zwischen verschiedenen Proben eine annähernd gleiche Konzentration an Nukleinsäuren mit möglichst geringen Konzentrationsschwankungen aufweist.

Darüber hinaus ist es wünschenswert, dass die aufgereinigten Nukleinsäuren unmittelbar für die weiteren Anwendungen einsatzfertig sind, d.h. bspw. keine weiteren Umpufferungen oder ähnliches erforderlich sind. Dies ist basierend auf den im Stand der Technik bekannten Aufreinigungsverfahren nicht immer möglich, da diese zur Elution der gereinigten Nukleinsäuren oft hohe pH-Werte und/oder hohe Salzkonzentrationen erforderlich machen. Daher ist es oftmals notwendig, die aufgereinigte Nukleinsäure zuvor zu fällen oder aber beispielsweise den pH-Wert für die nachfolgenden Anwendungen (downstream Applikationen) einzustellen, wodurch sich jedoch auch wieder die Konzentration im Eluat verändern kann.

Auch wenn die bekannten Verfahren zur Aufreinigung von Nukleinsäuren und mit gewissen Einschränkungen auch zur Aufreinigung einer definierten Menge von Nukleinsäuren geeignet sind, besteht das Bedürfnis, die bestehenden Verfahren zu verbessern, insbesondere die Aufreinigung einer definierten Menge an Nukleinsäuren aus verschiedenen Proben zu ermöglichen, bei der nur geringe Schwankungen in der Konzentration der aufgereinigten Nukleinsäure auftreten.

Der vorliegenden Erfindung liegt daher die Aufgabe zugrunde, die bestehenden Verfahren zur entsprechenden Aufreinigung von Nukleinsäuren zu verbessern.

Die vorliegende Erfindung löst diese Aufgabe mit einem Verfahren zur Isolierung und/oder Aufreinigung einer definierten Menge von Nukleinsäuren aus einer nukleinsäurehaltigen Probe, das wenigstens die nachfolgenden Schritte aufweist:
a. Inkontaktbringen der nukleinsäurehaltigen Probe mit einer definierten Menge einer nukleinsäurebindenden Phase mit folgenden Merkmalen:
   (i) die nukleinsäurebindende Phase weist nukleinsäurebindende Liganden auf, die wenigstens eine protonierbare Gruppe aufweisen;
   (ii) die nukleinsäurebindenden Liganden liegen an einen Träger gebunden vor, die nukleinsäurebindende Phase weist eine Oberfläche mit einer niedrigen Ladungsdichte auf, wobei die niedrige Ladungsdichte durch eines oder mehrere der nachfolgenden Merkmale erzielt wird:
      aa) die nukleinsäurebindenden Liganden weisen am Träger gebunden jeweils nicht mehr als ein oder zwei protonierbare Gruppen zur Bindung der Nukleinsäuren auf; und/oder
      bb) die nukleinsäurebindenden Liganden sind ausgewählt aus der Gruppe der Mono- und Diamine; und/oder
      cc) der Träger ist mit einer Mischung aus nukleinsäurebindenden Liganden und Verdünnungsliganden beschichtet, wobei die Verdünnungsliganden keine Nukleinsäuren binden oder die Nukleinsäuren mit einer geringeren Affinität als die nukleinsäurebindenen Liganden binden, und wobei der Anteil der nukleinsäurebindenen Liganden im Verhältnis zu den Verdünnungsliganden ≤ 50% beträgt,
   wobei die Menge an Nukleinsäuren in der Probe die Bindungskapazität der eingesetzten nukleinsäurebindender Phase übersteigt;
b. Bindung der Nukleinsäuren an die nukleinsäurebindende Phase bei einem pH-Wert (Bindungs pH-Wert), der unterhalb des pKs-Wertes wenigstens einer der protonierbaren Gruppen liegt;
c. Elution der Nukleinsäuren bei einem pH-Wert, der oberhalb des Bindungs pH-Wertes liegt, wobei eine definierte Menge an Nukleinsäuren erhalten wird.

Die vorliegende Erfindung betrifft die Isolierung und/oder Aufreinigung von Nukleinsäuren mittels einer speziellen nukleinsäurebindenden Phase, welche die Aufreinigung/Isolierung einer definierten Menge an Nukleinsäuren aus einer Probe mit geringen Konzentrationsschwankungen erlaubt. Entscheidend hierfür ist die Ausgestaltung der nukleinsäurebindenden Phase. Zur Bindung der Nukleinsäuren aus der Probe weist diese nukleinsäurebindende Liganden auf, die wenigstens eine protonierbare Gruppe aufweisen. Geeignete protonierbare Gruppen, insbesondere Aminogruppen, sind nachfolgend im Detail beschrieben. Die Bindung der Nukleinsäuren erfolgt bei einem pH-Wert unterhalb des pKs-Wertes wenigstens einer dieser protonierbaren Gruppen. Die protonierbaren Gruppen nehmen ein oder mehrere Protonen auf und werden dadurch positiv geladen, was dazu führt, dass die nukleinsäurebindende Phase die negativ geladenen Nukleinsäuren binden kann. Die Elution erfolgt bei einem höheren pH-Wert, wodurch die positive Ladung der protonierbaren Gruppe geringer und die an die nukleinsäurebindende Phase gebundene Nukleinsäure freigesetzt wird. Gemäß einigen Ausführungsformen kann die protonierbare Gruppe bei der Elution sogar neutral oder ggf. auch negativ geladen sein. Das Verfahren kann zur Isolierung und/oder Aufreinigung von Nukleinsäuren aus einer biologischen Probe eingesetzt werden. Darüber hinaus kann das Verfahren im Anschluss an ein klassisches Nukleinsäureaufreinigungsverfahren durchgeführt werden, um bspw. bereits aufgereinigte Nukleinsäuren zu normalisieren und so eine definierte Menge an Nukleinsäuren zu erhalten.

Ein wesentliches Merkmal der vorliegenden Erfindung ist die Oberfläche der nukleinsäurebindenden Phase. Diese weist erfindungsgemäß eine niedrige Ladungsdichte auf. Diese spezielle Oberfläche hat in Kombination mit dem Merkmal, dass die Menge an Nukleinsäuren in der Probe die Bindungskapazität der eingesetzten Menge an nukleinsäurebindender Phase übersteigt, überraschender Weise den Effekt, dass eine definierte Menge an Nukleinsäuren aus der Probe isoliert werden kann, wobei im Vergleich mit dem Stand der Technik deutlich geringere Schwankungen in der Nukleinsäurekonzentration auftreten.

Im Unterschied zum Stand der Technik wird erfindungsgemäß nicht versucht, eine Oberfläche zu erzielen, welche die Bindung einer besonders großen Menge an Nukleinsäuren erlaubt. Vielmehr wird bewusst die Ladungsdichte auf der Oberfläche und damit die Nukleinsäurebindungskapazität der nukleinsäurebindenden Phase verringert. Daher kann nur eine begrenzte, aber für eine bestimmte Probe immer gleiche d.h. definierte Menge an Nukleinsäuren gebunden und entsprechend eluiert werden. Für ein bestimmtes biologisches Probenmaterial muss daher nur in vorteilhafter Weise einmalig die Bindekapazität der nukleinsäurebindenden Phase bestimmt werden. Weitere Quantifizierungsschritte können entfallen, da eine bestimmte Menge an nukleinsäurebindender Phase nur eine definierte und daher immer gleiche Nukleinsäuremenge binden kann. Dadurch können in vorteilhafter Weise Arbeitsschritte - wie bspw. das Aufkonzentrieren oder das Verdünnen der aufgereinigten Proben entfallen, die andernfalls notwendig wären, um alle Proben auf die gleiche Konzentration an gereinigten Nukleinsäuren zu bringen.

Der Begriff "eine definierte Menge" bezieht sich insbesondere auf eine Konzentration an Nukleinsäuren, die innerhalb eines definierten, d.h. engen Schwankungsbereichs liegt. Wenn dieser für eine bestimmte Probe bzw. Probenmaterial nicht bekannt ist, kann dieser Bereich (experimentell) bestimmt werden. Vorzugsweise liegen die mit dem erfindungsgemäßen Verfahren erzielten Nukleinsäuremengen bzw. Konzentrationen in einem engen Bereich, so dass die Schwankungen je nach Probenmaterial bei weniger als +/- 30 %, vorzugsweise weniger als +/- 20 % und insbesondere in einem Bereich von +/- 15 % und besonders bevorzugt +/- 10 % liegen.

Die Menge der nukleinsäurebindenden Phase im Verhältnis zu der Menge/Konzentration der Nukleinsäuren im Probenmaterial sollte vorzugsweise so gewählt sein, dass man immer im Plateaubereich und somit im Sättigungsbereich der Nukleinsäurebindekapazität der nukleinsäurebindenden Phase liegt. Daher wird die Menge an nukleinsäurebindender Phase (bspw. die Menge an Partikeln oder die Größe der mit nukleinsäurebindenden Liganden beschichteten Trägerfläche) so gewählt, dass die Nukleinsäuren in der Probe im Überschuss zu der Nukleinsäurebindekapazität der eingesetzten Menge an nukleinsäurebindender Phase vorliegen. Aufgrund der geringen Nukleinsäurebindekapazität der erfindungsgemäßen nukleinsäurebindenden Phase hat das erfindungsgemäße Verfahren deutliche Vorteile gegenüber dem Einsatz von nukleinsäurebindenden Phasen/Partikeln, die eine hohe Nukleinsäurebindekapazität aufweisen (bspw. Silikapartikel oder auf herkömmliche Weise mit Polyaminen beschichtete Partikel). Im Stand der Technik muss man entweder hohe Schwankungen in der Ausbeute an Nukleinsäuren in Kauf nehmen oder derart geringe Mengen an nukleinsäurebindender Phase/Partikel einsetzen, dass die Handhabung insbesondere im Rahmen eines automatisierten Prozesses erschwert wird. So müsste bspw. bei Einsatz der im Stand der Technik bekannten Partikel die eingesetzte Menge derart herunterverdünnt werden, dass sie nur schwierig in einem automatisierten Verfahren zu handhaben wären. Dies birgt wiederum Fehlerquellen, da der Verlust von bereits wenigen Partikeln mit hoher Nukleinsäurebindekapazität eine deutliche Schwankung in der Ausbeute an Nukleinsäuren zur Folge hätte. Auch unter diesen Gesichtspunkten ist das erfindungsgemäße Verfahren daher dem Stand der Technik überlegen.

Zur Aufreinigung einer definierten Menge an Nukleinsäuren und damit Normalisierung der Nukleinsäurekonzentration wird erfindungsgemäß der nukleinsäurebindenden Phase so viel Nukleinsäure angeboten, dass die Nukleinsäurebindekapazität der eingesetzten nukleinsäurebindenden Phase erschöpft ist. Der restliche, ungebundene Teil der Nukleinsäure wird bei der Aufreinigung verworfen. Optional kann die nukleinsäurebindende Phase mit der gebundenen Nukleinsäure vor der Elution gewaschen werden. Die Nukleinsäure wird im Elutionsschritt von der Oberfläche der nukleinsäurebindenden Phase in die Elutionslösung überführt. Dadurch wird für ein gegebenes Probenmaterial immer die gleiche, d.h. definierte Menge an Nukleinsäure bei der Aufreinigung erhalten.

Im Verfahren der Erfindung wird die Oberfläche mit niedriger Ladungsdichte durch eines oder mehrere der nachfolgenden Merkmale erzielt:
a. die nukleinsäurebindenden Liganden weisen am Träger gebunden jeweils nicht mehr als ein oder zwei protonierbare Gruppen zur Bindung der Nukleinsäuren auf; und/oder
b. die nukleinsäurebindenden Liganden sind ausgewählt aus der Gruppe der Mono- und Diamine; und/oder
c. der Träger ist mit einer Mischung aus nukleinsäurebindenden Liganden und Verdünnungsliganden beschichtet, wie im Anspruch 1 definiert.

Offenbart wird, dass die nukleinsäurebindenden Liganden ' im Unterschuss an den Träger gebunden vorliegen können.

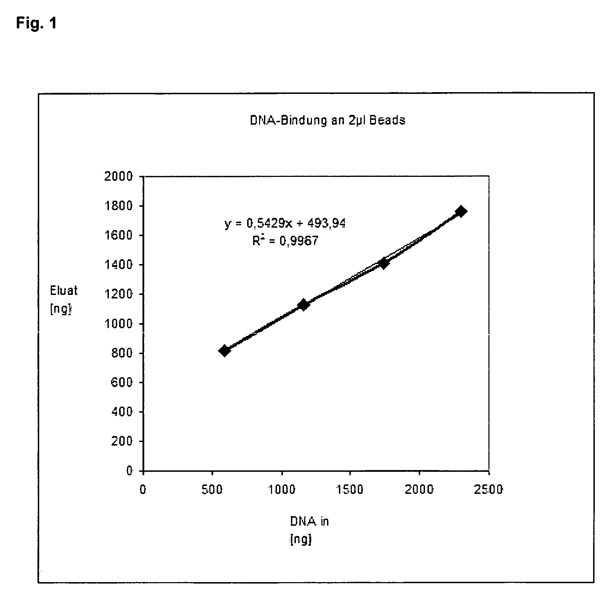

Erfindungsgemäß wird wie ausgeführt eine nukleinsäurebindende Phase eingesetzt, die eine Oberfläche mit niedriger Ladungsdichte aufweist. Hierfür weisen die nukleinsäurebindenden Liganden vorzugsweise jeweils nicht mehr als ein oder zwei protonierbare Gruppen wie bspw. Amino-Gruppen zur Bindung der Nukleinsäuren auf. Entsprechend sind die nukleinsäurebindenden Liganden vorzugsweise ausgewählt aus der Gruppe der Mono- und Diamine. Es hat sich gezeigt, dass im Gegensatz zu einer herkömmlichen Beschichtung mit Polyaminen wie Spermin und Spermidin Mono- und/oder Diamine sehr gut zur Normalisierung von Nukleinsäurekonzentrationen geeignet sind. Auf herkömmliche Weise mit Polyaminen beschichtete Träger weisen augenscheinlich eine zu große Ladungsdichte auf der Oberfläche auf, um für eine Normalisierung geeignet zu sein. Sie binden daher größere Mengen an Nukleinsäuren. Wird solch eine Oberfläche mit einer nukleinsäurehaltigen Probe in Kontakt gebracht, findet man eine starke Abhängigkeit der Bindekapazität von der Nukleinsäurekonzentration. Typischerweise zeigt sich im untersuchten Bereich ein proportionales Verhältnis zwischen angebotener und eluierter DNA-Menge. Fig. 1 zeigt am Beispiel von mit dem Polyamin Spermin beschichteten Polymerpartikeln dieses Verhalten, was die beschriebenen Schankungen in der Menge an aufgereinigter Nukleinsäure zur Folge hat und wodurch eine Normalisierung bei der Aufreinigung nicht möglich ist. Die bevorzugt eingesetzten Mono- und/oder Diamine können entweder einzeln oder als Gemisch aufgebracht werden.

Durch die Verwendung von vorzugsweise Mono- und/oder Diaminen bei der Beschichtung des Trägers erzeugt man dagegen eine begrenzte, aber definierte Mengen an Ladungsträgern und damit nukleinsäurebindenden Gruppen auf der Oberfläche. Die so erzeugte Ladungsträgerdichte zur Bindung der Nukleinsäuren ist deutlich geringer als bei einer entsprechenden Beschichtung mit Polyaminen, wie z. B. Spermin oder gar Polyethyleniminen. Insbesondere mit N-Proply-1,3-Propandiaminen modifizierte Polymerpartikel sind gut geeignet, die Nukleinsäurekonzentration bereits bei der Aufreinigung zu normalisieren und damit eine definierte Menge an Nukleinsäuren aus einer Probe zu isolieren. Der Vorteil eines mit Monoaminen bzw. Diaminen beschichteten Trägers zur Herstellung einer nukleinsäurebindenden Phase liegt insbesondere darin, dass nur eine begrenzte, aber für eine bestimmte Probe innerhalb eines geringen Schwankungsbereichs gleiche Menge an Nukleinsäure gebunden wird. Durch die hohe Reproduzierbarkeit bei der Herstellung der Trägermaterialien und bei der Aminofunktionalisierung der Trägeroberfläche erreicht man eine enge Variabilität der Ladungsträgerdichte und damit der Nukleinsäurebindekapazität. Dadurch werden ungewünschte Schwankungen in der Nukleinsäurekonzentration bei der Aufreinigung vermieden. Die geschilderten Vorteile lassen sich nicht nur gegenüber Polyaminen aufzeigen, sondern zeigen sich insbesondere auch gegenüber den üblicherweise eingesetzten Silika-Partikeln, bei denen aufgrund der hohen Nukleinsäurebindekapazität der Oberfläche Schwankungen von bis zu 50 % bei der Isolierung der Nukleinsäuren aus gleichem Probenmaterial auftreten.

Überraschender Weise hat sich ferner gezeigt, dass eine niedrigere Ladungsdichte auf der Oberfläche des Trägers auch dadurch erreicht werden kann, dass sofern beispielsweise Polyamine als nukleinsäurebindende Liganden eingesetzt werden, der Träger nur mit einer geringen Menge an Polyaminen beschichtet wird, um die gewünschte geringe Ladungsdichte auf der Oberfläche des nukleinsäurebindenden Materials zu erhalten. Gemäß einer Ausführungsform werden die nukleinsäurebindenden Liganden daher beabstandet zueinander auf dem Trägermaterial angeordnet bzw. verdünnt. Um eine solche Anordnung der nukleinsäurebindenden Liganden zu erreichen, kann gemäß einer Ausführungsform der Träger nur mit geringen Mengen an nukleinsäurebindenden Liganden wie bspw. Polyaminen oder vorzugsweise Mono- und/oder Diaminen beschichtet werden. Vorzugsweise erfolgt die Funktionalisierung mit nukleinsäurebindenden Liganden daher im Unterschuss insbesondere in Relation zur Bindekapazität der Trägermaterialoberfläche. Dadurch wird quasi eine Verdünnung der nukleinsäurebindenden Liganden auf dem Träger erreicht, so dass weniger protonierbare Gruppen zur Verfügung stehen. Bspw. können nur ≤ 50%, ≤ 25%, ≤ 15%, ≤ 10% oder nur ≤ 5% der funktionellen bzw. mit den nukleinsäurebindenden Liganden funktionalisierbaren Gruppen auf dem Trägermaterial mit nukleinsäurebindenden Liganden funktionalisiert werden. Auch dadurch wird eine nukleinsäurebindende Phase mit einer geringen Ladungsdichte auf der Oberfläche erhalten.

Ferner kann die Beschichtung des Trägers mit einer Mischung aus nukleinsäurebindenden Liganden und sog. Verdünnungsliganden erfolgen. Der Begriff "Verdünnungsliganden" wird hier dazu verwendet, um die Funktion im Verhältnis zu den nukleinsäurebindenden Liganden darzustellen. Ihre Funktion ist es, die Menge an nukleinsäurebindenden Liganden auf dem Träger variieren und damit einstellen zu können und damit eine geringere Ladungsdichte auf der Oberfläche der nukleinsäurebindenden Phase einstellen zu können. Je höher der Anteil an Verdünnungsliganden im Verhältnis zu nukleinsäurebindenden Liganden ist, desto weniger nukleinsäurebindende Liganden werden auf den Träger aufgebracht und desto geringer ist die Nukleinsäurebindungskapazität. Die Verdünnungsliganden können negativ, positiv oder neutral geladen sein bzw. ionisierbare Gruppen aufweisen. Gemäß einer Ausführungsform binden die Verdünnungsliganden keine Nukleinsäuren oder sie binden die Nukleinsäuren mit einer geringen Affinität als die nukleinsäurebindenden Liganden. Sofern die Verdünnungsliganden Nukleinsäuren binden, binden sie diese gemäß einer Ausführungsform mit einer Affinität, die wenigstens 20%, wenigstens 50% oder wenigstens 75% unter der Affinität der nukleinsäurebindenden Liganden liegt. Der Anteil an nukleinsäurebindenden Liganden im Verhältnis zu den Verdünnungsgruppen kann bspw. ≤ 50%, ≤ 25%, ≤ 15%, ≤ 10% oder nur ≤ 5% betragen. Geeignete Verdünnungsliganden sind bspw. Dimethylamin, Diethylamin und Ammoniak oder Verdünnungsliganden aufweisend Gruppen der Formel R, -OH oder -OR, wobei R ein verzweigter oder unverzweigter Alkylrest ist. Ein weiteres Beispiel für einen geeigneten Verdünnungsliganden wäre Ethanolamin. Gemäß einer bevorzugten Ausführungsform wird eine Mischung aus nukleinsäurebindenden Liganden und Verdünnungsliganden zur Beschichtung des Trägers eingesetzt.

Die Bindekapazität der nukleinsäurebindenden Phase liegt gemäß einer Ausführungsform in einem Bereich von 1 bis 500µg Nukleinsäure pro mg nukleinsäurebindender Phase, wobei geringere Bindekapazitäten wie 1 bis 300µg, 1 bis 200µg, 10 bis 100µg Nukleinsäure pro mg nukleinsäurebindender Phase, insbesondere DNA, auch möglich sind. Gemäß einer bevorzugten Ausführungsform liegt die Bindekapazität der nukleinsäurebindenden Phase bei 0,01 bis 100µg, 0,025 bis 100µg, 0,05 bis 100µg oder 0,1 bis 100µg Nukleinsäure pro mg nukleinsäurebindender Phase, vorzugsweise bei 0,01 bis 50µg, 0,025 bis 50µg, 0,05 bis 50µg oder 0,1 bis 50µg Nukleinsäure pro mg nukleinsäurebindender Phase und besonders bevorzugt bei 0,01 bis 10µg, 0,025 bis 10µg, 0,05 bis 10µg oder 0,1 bis 10µg Nukleinsäure pro mg nukleinsäurebindender Phase. Derartige nukleinsäurebindende Phasen sind insbesondere geeignet, um auch geringere Mengen an Nukleinsäuren zu normalisieren. Dies insbesondere, wenn mit kleinen Volumen hantiert wird.

Um eine schonende Elution der Nukleinsäuren zu erreichen, erfolgt die Elution vorzugsweise bei einem pH-Wert, der oberhalb des Bindungs pH-Wertes liegt, jedoch wenigstens eine pH-Einheit, vorzugsweise wenigstens zwei pH-Einheiten unterhalb des pKs-Wertes wenigstens einer der protonierbaren Gruppen, vorzugsweise aller protonierbarer Gruppen liegt. Dies hat den erheblichen Vorteil, dass die Elution auch bei schonenden Bedingungen durchgeführt werden kann. Die vorliegende Erfindung erlaubt daher die Elution bei einem pH-Wert, der unterhalb der pKs-Werte der protonierbaren Gruppen liegt.

Gemäß einer Ausführungsform der vorliegenden Erfindung erfolgt die Bindung der Nukleinsäuren bei einem pH-Wert von 3 bis 8. Diese Angabe bezieht sich auf den pH-Wert während der Bindung und damit in der Probe. Das erfindungsgemäße Verfahren ist je nach Gestaltung der festen Phase auch bei sehr schonenden Bedingungen durchführbar, so dass die Bindung der Nukleinsäuren auch bei einem pH-Wert von 4 bis 7,5; vorzugsweise bei 5 bis 7,5; besonders bevorzugt bei 5 bis 7 und ganz besonders bevorzugt bei 6,5 bis 7 und damit im nahezu neutralen Bereich durchgeführt werden kann. Aufgrund der Tatsache, dass die protonierbaren Gruppen vorzugsweise einen pKs-Wert von 9 bis 12, weiter bevorzugt von 10 bis 12 aufweisen, liegen diese selbst bei relativ neutralen pH-Werten ausreichend positiv geladen vor, um die effektive Anbindung der Nukleinsäuren zu erlauben. Daher kann die Bindung unter sehr schonenden Bedingungen erfolgen, was eine Schädigung der Nukleinsäuren verhindert.

Darüber hinaus hat es sich als vorteilhaft erwiesen, die Bindung bei einer niedrigen Salzkonzentration vorzunehmen. Gemäß einer Ausführungsform liegt daher die Salzkonzentration bei der Bindung der Nukleinsäuren an die nukleinsäurebindende Phase bei ≤ 1 M. Vorzugsweise liegt die Salzkonzentration bei ≤ 0,5 M, ≤ 0,25 M oder sogar ≤ 0,1 M. Eine niedrige Salzkonzentration ist bevorzugt, um die Bindung der Nukleinsäuren an die feste Phase zu optimieren. Zu hohe Ionenkonzentrationen können die ionischen Wechselwirkungen von Nukleinsäure und der nukleinsäurebindenden Phase negativ beeinflussen. Es hat sich herausgestellt, dass der Bindungspuffer auch gewisse Mengen organische Substanzen wie bspw. Kohlenhydrate, Alkohole (wie bspw. Ethanol, Methanol), Aceton, Acetonitril oder Mischungen derselben enthalten kann. Diese beeinträchtigen die Bindung nicht.

Ein weiterer wesentlicher Schritt des vorliegenden Verfahrens ist die Elution der Nukleinsäuren. Wie dargelegt, erfolgt die Nukleinsäurefreisetzung bei einem pH-Wert, der oberhalb des Bindungs pH-Wertes liegt. Dies hat zur Folge, dass die protonierbaren Gruppen bei der Elution weniger positiv geladen vorliegen, wodurch die Freisetzung der Nukleinsäuren begünstigt wird. Darüber hinaus liegt der pH-Wert bei der Elution vorzugsweise wenigstens eine pH-Einheit unterhalb des pKs-Wertes wenigstens einer der protonierbaren Gruppen der nukleinsäurebindenden Phase. Dies hat wie oben dargelegt zur Folge, dass die Elution unter besonders schonenden Bedingungen durchgeführt werden kann.

Vorzugsweise erfolgt die Elution je nach eingesetzten nukleinsäurebindenden Liganden bzw. nukleinsäurebindenden Phase bei einem pH-Wert von 7,5 bis 11, von 7,5 bis 10, vorzugsweise bei einem pH-Wert von 8 bis 9 bzw. 8,2 bis 8,8. Bei diesen pH-Werten werden besonders vorteilhafte Ergebnisse erzielt, da die Nukleinsäuren besonders schonend freigesetzt werden.

Um die direkte Weiterverarbeitung der isolierten Nukleinsäuren in der Elutionslösung, vorzugsweise in einem Elutionspuffer zu ermöglichen, weist diese vorzugsweise eine niedrige Salzkonzentration auf. Die Salzkonzentration liegt daher gemäß einer Ausführungsform bei ≤ 1 M vorzugsweise bei ≤ 0,5 M, bei ≤ 0,25 M, bei ≤ 0,1 M, besonders bevorzugt bei ≤ 50 mM, ≤ 25 mM oder sogar ≤ 10 mM. Geeignete Salze können Halogenide, insbesondere Chloride, der Alkali und Erdalkalimetalle oder Ammonium, andere Salze von Mineralsäuren, Acetate, Borate sowie Verbindungen wie Tris, Bis-Tris sowie auf der Basis organischer Puffer, wie bspw. MES, CHAPS, HEPES und ähnliche darstellen. Geeignete Substanzen zur Elution sind darüber hinaus im Stand der Technik bekannt.

Insbesondere bei der vakuum- oder zentrifugationsbasierten Elution der Nukleinsäuren bspw. bei Einsatz einer nukleinsäurebindenden Membran oder bei Einsatz von nukleinsäurebindenden Partikeln in Kombination mit einer Membran, welche als Barriere für die Partikel dient, kann es insbesondere bei kleinen Elutionsvolumina von Vorteil sein, wenn zusätzliche Maßnahmen ergriffen werden, die sicherstellen, dass innerhalb geringer Schwankungen auch jeweils das gleiche Elutionsvolumen erzielt wird. Dies insbesondere wenn mit Mehrfachansätzen wie bspw. einer 48 oder 96er Platte gearbeitet wird. So hat es sich als vorteilhaft erwiesen, auf die Elutionslösung wenigstens einen Kohlenwasserstoff zu geben. Dadurch wird in vorteilhafter Weise ein gleichmäßigeres Eluatvolumen erhalten. Dies ist vermutlich darauf zurückzuführen, dass der aufgeschichtete Kohlenwasserstoff den vollständigen Durchtritt der Elutionslösung durch die Membran fördert, wodurch ein gleichmäßigeres Eluatvolumen erhalten wird. Der Kohlenwasserstoff und/oder das Gemisch aus Kohlenwasserstoffen, können eines oder mehrere der nachfolgenden Merkmale aufweisen:
a) der Kohlenwasserstoff ist ein unsubstituiertes oder substituiertes Alkan;
b) der Kohlenwasserstoff ist ein mit Wasser nicht mischbares Alkan;
c) der Kohlenwasserstoff ist ein acyclisches Alkan;
d) der Kohlenwasserstoff ist ein unverzweigtes acyclisches Alkan;
e) der Kohlenwasserstoff ist ein verzweigtetes acyclisches Alkan;
f) der Kohlenwasserstoff ist ein cyclisches Alkan;
g) der Kohlenwasserstoff ist ein Alkan mit 6 bis 16 Kohlenstoffatomen;
h) der Kohlenwasserstoff ist ein Alkan mit 8 bis 12 Kohlenstoffatomen;
i) der Kohlenwasserstoff ist ein Alkan, das ausgewählt ist aus der Gruppe n-Octoan, n-Nonan, n-Decan und n-Dodecan; und/oder
j) der Kohlenwasserstoff ist vorzugsweise ein Mineralöl.

Um die Aufreinigung zu begünstigen, wird nach der Bindung und vor der Elution der Nukleinsäuren vorzugsweise wenigstens ein Waschschritt durchgeführt. Zum Waschen sind wässrige Lösungen mit niedrigen Salzkonzentrationen und aber auch Wasser bevorzugt. Wenn Salze in den Waschpuffern bzw. Waschlösungen enthalten sind, so bevorzugt in einer Konzentration von ≤ 400 mM, besonders bevorzugt von ≤ 200 mM, ≤ 100 mM, ≤ 50 mM und/oder sogar ≤ 25 mM. Organische Bestandteile können in dem Waschpuffer enthalten sein, so bspw. Alkohole, Polyole, Polyethylenglykole, Aceton, Acetonitril, Kohlenhydrate oder Mischungen derselben. Die Waschpuffer bzw. Waschlösungen können jedoch ohne störende Mengen der entsprechenden organischen Bestandteile vorliegen, so dass nachfolgende Anwendungen, wie bspw. enzymatische Verarbeitungen, Amplifikationsreaktionen und dergleichen ("downstream" Applikationen) nicht behindert werden.

Die nukleinsäurebindenden Liganden werden mit dem Träger kovalent oder nichtkovalent durch elektrostatische, polare oder hydrophobe Wechselwirkungen verknüpft. Vorzugsweise erfolgt dies im Rahmen einer Beschichtung. Sofern das Trägermaterial keine geeigneten funktionellen Gruppen zur Anbindung der nukleinsäurebindenden Liganden aufweist, kann zunächst eine Funktionalisierung des Trägermaterials erfolgen, um dieses mit geeigneten funktionellen Gruppen, insbesondere Carboxygruppen, auszustatten. Hierbei kann das entsprechende Profil der Beschichtung des Trägermaterials mit nukleinsäurebindenden Liganden im Unterschuss auch dadurch erzielt werden, dass das Trägermaterial entsprechend mit weniger funktionellen Gruppen zur Anbindung der nukleinsäurebindenden Liganden ausgestattet wird. Geeignete Beschichtungsverfahren sind im Stand der Technik bekannt und bedürfen daher keiner näheren Beschreibung. Die Bindung der nukleinsäurebindenden Liganden an den Träger und insbesondere an die funktionellen Gruppen des Trägers kann auf verschiedene Arten erfolgen. Die nukleinsäurebindenden Liganden können bspw. im Fall von Di - oder Polyaminen über eine Aminogruppe verbunden werden. Die nukleinsäurebindenden Liganden können jedoch auch eine funktionelle Gruppe aufweisen, die zur Anbindung an den Träger genutzt wird. Geeignete funktionelle Gruppen sind im Stand der Technik bekannt. Ferner besteht auch die Möglichkeit, die nukleinsäurebindenden Liganden direkt mit der Oberfläche des Trägers, vorzugsweise eines Polymerträgers zu verknüpfen. Dies kann bspw. direkt über das Kohlenstoffgerüst erfolgen.

Bevorzugte protonierbare Gruppen, die sich für die Bindung von Nukleinsäuren bewährt haben, sind Aminogruppen, bevorzugt sind primäre, sekundäre und tertiäre Aminogruppen. Vorzugsweise weisen die protonierbaren Gruppen, wie insbesondere die Aminogruppen einen pKs-Wert von wenigstens 8 auf. Bevorzugt liegt der pKs-Wert bei 9 bis 12, vorzugsweise bei 10 bis 12. Die nukleinsäurebindenden Liganden weisen vorzugsweise 1 bis 2 Aminogruppen auf. Bevorzugte nukleinsäurebindende Liganden sind bspw. primäre, sekundäre und tertiäre Mono- und Diamine. Diese können substituiert oder unsubstituiert sein.

Bevorzugte Beispiele für nukleinsäurebindende Liganden sind insbesondere primäre, sekundäre und tertiäre Amine der Formeln
(a) R₃N
(b) R₂NH
(c) RNH₂ und/oder
(d) X-(CH₂)ₙ-Y mit X = R₂N, RNH oder NH₂ und Y = R₂N oder RNH oder NH₂
wobei
R = unabhängig voneinander ein verzweigter, unverzweigter oder ringförmiger Alkyl-, Alkenyl-, Alkinyl- oder aromatischer Substituent ist, wobei dieser auch ein oder mehrere Heteroatome enthalten kann;
n= 0 bis 20

Bevorzugt wird N-Propyl-1,3-propandiamin als nukleinsäurebindender Ligand eingesetzt.

Darüber hinaus können auch ringförmige Amine, aromatische Amine oder aminofunktionalisierte Heterozyklen eingesetzt werden. Die Amine können Substituenten tragen, bspw. Alkyl-, Alkenyl-, Alkinyl- oder aromatische Substituenten, darüber hinaus können die Kohlenwasserstoffketten auch zu einem Ring geschlossen sein. Die Kohlenwasserstoffketten können auch Heteroatome, wie Sauerstoff, Stickstoff, Schwefel oder Silizium, oder Verzweigungen aufweisen. Die Aminogruppen der Amine weisen vorzugsweise pKs-Werte von 9 bis 12, besonders bevorzugt von 10 bis 12 auf.

Weitere geeignete nukleinsäurebindende Liganden sind Polyoxyalkylenamine mit einer, zwei oder drei Aminogruppen. Diese sind beispielsweise erhältlich unter dem Namen "Jeffamine" Polyoxylalkylenamine. Jeffamine enthalten primäre Aminogruppen, die an den Terminus des Polyetherbackbones gebunden sind. Das Polyetherbackbone kann auf Propylenoxid, Ethylenoxid oder Mischungen daraus basieren; auch ist der Einsatz anderer Backbonesegmente denkbar.

Auch Mischungen der entsprechenden nukleinsäurebindenden Liganden können erfindungsgemäß eingesetzt bzw. auf einen Träger aufgebracht werden.

Vorzugsweise stehen die Aminogruppen der nukleinsäurebindenden Liganden nicht in Konjugation mit einer die Elektronendichte verringernden Gruppe wie bspw. einer Carboxylgruppe, einer Carbonylgruppe, einer Gruppe mit C-C-Doppelbindungen oder einer ß-Hydroxyethylgruppe, so dass ihr pKs-Wert vorzugsweise zwischen 9 und 12 liegt. Eine Konjugation mit einer Elektronendichte verringernden Gruppe gilt dann als vorhanden, wenn eine Aminofunktion und die entsprechende, die Elektronendichte verringernde Gruppe über nur drei, zwei oder weniger Kohlenstoffatome verbunden sind.

Als Träger für die nukleinsäurebindenden Liganden kommen bspw. organische Polymere wie Polystyrol und seine Derivate, Polyacrylate und -methacrylate, und ihre Derivate oder Polyurethane, Nylon, Polyethylen, Polypropylen, Polybutylen, und Copolymere aus diesen Materialien infrage. Darüber hinaus können diese nukleinsäurebindenden Liganden auch mit Polysacchariden, insbesondere Hydrogelen wie Agarose, Cellulose, Dextran, Sephadex, Sephacryl, Chitosan verknüpft sein. Des weiteren können die nukleinsäurebindenden Liganden auch an anorganische Träger wie bspw. Glas oder weitere Metall- und Halbmetalloxide (insbesondere Oxide der Formel MeO, wobei Me vorzugsweise ausgewählt ist aus der Gruppe umfassend Al, Ti, Zr, Si, B, insbesondere Al₂O₃, TiO₂, Silica und Boroxid) oder Metalloberflächen, wie z.B. Gold, angebunden werden. Vorzugsweise weist das Trägermaterial keine eigene Nukleinsäurebindekapazität auf.

Magnetische Partikel sind besonders vorteilhaft in der Handhabung. Die nukleinsäurebindende Phase ist daher vorzugsweise magnetisch und kann paramagnetisch, ferrimagnetisch, ferromagnetisch oder superparamagnetisch sein. Bevorzugt sind superparamagnetische oder paramagnetische Partikel. Die einzelnen Schritte und Ausführungsvarianten zur Handhabung von magnetischen Partikeln wie sie erfindungsgemäß bevorzugt zum Einsatz kommen, sind im Stand der Technik wohl bekannt und benötigen daher ebenfalls keine genaue Beschreibung.

Die nukleinsäurebindenden Liganden können direkt oder aber über Abstandshalter, sogenannte "Spacer", an diese Träger gebunden sein. Sie können auch Teil eines größeren Moleküls sein. Beispiele für Spacer sind Kohlenwasserstoffketten, Poylethylen- oder Polypropylenglykole, sowie funktionalisierte Silane. Diese Spacer können verzweigt oder unverzweigt vorliegen.

Als chemische Funktionalitäten für die Anbindung der nukleinsäurebindenden Liganden können Säureamide oder - anhydride, Epoxide, Tosylgruppen, Formylgruppen, Sulfonylchloride, Maleinimide oder mit Carbodiimidchemie aktivierte Carboxylatgruppen eingesetzt werden. Im Rahmen der Erfindung ebenfalls möglich ist eine nichtkovalente Anbindung der nukleinsäurebindenden Liganden wie bspw. Amine, z.B. über ionische Wechselwirkungen oder über adsorptive Prozesse. Die nukleinsäurebindenden Liganden können auch über Thiole an z.B. Goldoberflächen angebunden sein. Bevorzugt ist die Anbindung der nukleinsäurebindenden Liganden an carboxylierte Oberflächen.

Weitere Ausführungsformen von geeigneten Trägermaterialien zur Anbindung der nukleinsäurebindenden Liganden umfassen nichtmagnetische und magnetische Partikel, Säulenmaterialien, Membranen, sowie Oberflächenbeschichtungen. Ferner seien Träger wie Röhrchen, Membrane, Vliese, Papier, Reaktionsgefäße wie PCR-Gefäße, "Eppendorf-Tubes", Multiplates, Chips und Mikroarrays genannt. Diese Träger können wie oben beschrieben mit den nukleinsäurebindenden Liganden beschichtet werden, um eine erfindungsgemäße nukleinsäurebindende Phase zu erhalten. Durch die Dichte und Größe der mit nukleinsäurebindenden Liganden beschichteten Fläche eines solchen Trägermaterials wird wiederum eine definierte Menge einer nukleinsäurebindenden Phase für die Aufreinigung zur Verfügung gestellt. Bspw. können einzelne oder alle Reaktionsbereiche (Wells) einer Mikrotiterplatte oder eines Multiplates oder einer entsprechenden Vorrichtung zur Prozessierung von Proben ganz oder teilweise mit den nukleinsäurebindenden Liganden beschichtet werden, um eine nukleinsäurebindende Phase zu erhalten.

Besonders entscheidend für den Wirkungsgrad der Nukleinsäureaufreinigung ist eine effiziente Elution und damit Ablösung der gebundenen Nukleinsäuren von der nukleinsäurebindenden Phase. Hier wurde überraschend festgestellt, dass nicht nur die pKs-Werte der protonierbaren Gruppen der nukleinsäurebindenden Liganden entscheidend sind. Auch die Struktur der nukleinsäurebindenden Phase und die Anwesenheit anderer funktioneller Gruppen tragen dazu bei, die Elution bei pH-Werten im neutralen bzw. schwach alkalischen Bereich zu begünstigen und zu verbessern.

Gemäß einer Ausführungsform trägt die nukleinsäurebindende Phase zusätzlich funktionelle Gruppen, die beim Elutions pH-Wert die Elution der Nukleinsäuren fördern, bspw. indem sie beim Elutions pH-Wert einen abstoßenden Effekt ausüben. Vorzugsweise liegen diese funktionellen Gruppen daher bei der Elution negativ geladen vor. Die pKs-Werte dieser Gruppen können bspw. in einem Bereich von 0 bis 7, vorzugsweise 1 bis 5 liegen. Geeignet sind bspw. Ionenaustauscher, insbesondere Kationenaustauscher, bevorzugt saure Gruppen wie bspw. Carboxylgruppen. Weitere geeignete Gruppen sind Betaine, Sulfonate, Phosphonate und Phosphate. Bspw. kann der feste Träger mit Carboxylgruppen funktionalisiert sein, um eine Anbindung der nukleinsäurebindenden Liganden zu ermöglichen. Bei der Anbindung der nukleinsäurebindenden Liganden kann die Konzentration selbiger so gewählt sein, dass einige der Carboxylgruppen frei vorliegen und daher nicht mit den nukleinsäurebindenden Liganden funktionalisiert werden. Diese behindern bei niedrigen pH-Werten die Bindung der Nukleinsäuren nicht. Bei höheren pH-Werten liegen diese jedoch vorzugsweise negativ geladen vor und begünstigen dadurch die Ablösung der Nukleinsäuren von den nukleinsäurebindenden Liganden. Diese Wechselwirkung kann ferner durch die Auswahl der Länge bzw. des Abstandes zwischen den protonierbaren Gruppen der nukleinsäurebindenden Liganden und den negativ ionisierbaren Gruppen, wie bspw. den Carboxylgruppen begünstigt werden. Dadurch wird in vorteilhafter Weise die Elution bei niedrigen pH-Werten begünstigt, so dass die Ausbeute steigt. Die Auswahl, Stärke und Länge der funktionellen Gruppen, die bei dem Elutions pH-Wert einen abstoßenden Effekt auf die Nukleinsäuren ausüben, variiert je nach ausgewählter nukleinsäurebindender Gruppe, so insbesondere der Anzahl der protonierbaren Gruppen pro nukleinsäurebindender Gruppe und deren Abstand zu den die Elution fördernden funktionellen Gruppen. Gemäß einer Ausführungsform fungieren die die Elution fördernden funktionellen Gruppen als Verdünnungsliganden.

Durch Wahl/Kombination der beschriebenen Parameter, insbesondere der die Elution fördernden funktionellen Gruppen, der Verdünnungsliganden, sowie der Verdünnung bzw. Mischung mit nukleinsäurebindenden Liganden kann der pH-Wert der nukleinsäurebindenden Phase in Bezug auf die Elutionsbedingungen optimal eingestellt werden. Entsprechend kann das Elutionsprofil der nukleinsäurebindenden Phase, insbesondere der Elutions pH-Wert gesteuert bzw. eingestellt werden.

Nukleinsäuren, die mit dem erfindungsgemäßen Verfahren aufgereinigt werden können, können in Körperflüssigkeiten wie Blut, Urin, Stuhl, Speichel, Sputum, oder sonstigen Körperflüssigkeiten, in biologischen Quellen wie Gewebe, Zellen, insbesondere Tierzellen, Humanzellen, Pflanzenzellen, Bakterienzellen und dergleichen, Organen wie Leber, Niere oder Lunge etc. vorliegen. Dazu kann die Nukleinsäure aus Trägermaterialien wie Swabs, PapSmears, sowie stabilisierenden Medien wie PreServCyt oder Surepath, oder auch aus weiteren Flüssigkeiten, wie z.B. Säften, wässrigen Proben oder allgemein Lebensmitteln gewonnen werden. Darüber hinaus können die Nukleinsäuren aus Pflanzenmaterial, bakteriellen Lysaten, in Paraffin eingebettetem Gewebe, wässrige Lösungen oder Gelen gewonnen werden.

Sofern die Nukleinsäure in einem Zellmaterial vorliegt, kann das Zellmaterial wie im Stand der Technik bekannt zerstört und insbesondere lysiert werden, um die Nukleinsäuren freizusetzen. Entsprechende Zellaufbruchsverfahren bzw. Lyseverfahren sind im Stand der Technik bekannt und benötigen daher keine weitere Beschreibung.

Aufgrund der Tatsache, dass man bei dem erfindungsgemäßen Verfahren auf Wasser basierende Puffer einsetzen und auf gefährliche Chemikalien verzichten kann, werden sehr saubere Eluate erzielt, die vorzugsweise keine Substanzen enthalten, die für die downstream Applikationen wie bspw. PCR, Restriktionsverdau, Transfektion oder Sequenzreaktionen nachteilig sind. Darüber hinaus ist es mit der erfindungsgemäßen Methode möglich, unter sehr schonenden Bedingungen, wie bspw. pH 6,5 - 7 die Nukleinsäure zu binden und nur bei leicht alkalischem pH wie 8,5 und sehr niedrigen Salzkonzentrationen wie zum Beispiel 25 mmol Tris zu eluieren, so dass für die weiteren Untersuchungen und Anwendungen (downstream Applikationen) keine Verdünnung oder Neutralisation notwendig ist.

Die erfindungsgemäß standardisierte bzw. normalisierte Nukleinsäurekonzentration kann bevorzugt für eine enzymatische Reaktion wie bspw. einer Nukleinsäureamplifikation, Modifikationsreaktion oder Sequenzierreaktionen eingesetzt werden. Die erfindungsgemäß aufgereinigten und damit normalisierten Nukleinsäuren eignen sich auch bspw. für Short Tandem Repeat (STR), Analysen und Transfektionen, bei denen ebenfalls definierte Mengen von Nukleinsäuren zum Einsatz kommen.

Darüber hinaus betrifft die vorliegende Erfindung die Verwendung einer nukleinsäurebindenden Phase wie oben beschrieben zur Aufreinigung einer definierten Menge von Nukleinsäuren aus einer Probe und damit zur Normalisierung der Nukleinsäurekonzentration. Die nukleinsäurebindende Phase weist nukleinsäurebindende Liganden mit wenigstens einer protonierbaren Gruppe auf, wobei die nukleinsäurebindenden Liganden an einen Träger gebunden vorliegen und die nukleinsäurebindende Phase eine Oberfläche mit einer niedrigen Ladungsdichte aufweist, wobei ein Elutions pH-Wert eingestellt wird, der oberhalb des Bindungs pH-Wertes liegt und wobei die Nukleinsäuren in der Probe im Überschuss zu der Bindungskapazität der eingesetzten Menge an nukleinsäurebindender Phase vorliegen.

Die niedrige Ladungsdichte wird durch eines oder mehrere der nachfolgenden Merkmale erzielt:
a) die nukleinsäurebindenden Liganden weisen am Träger gebunden jeweils nicht mehr als ein oder zwei protonierbare Gruppen zur Bindung der Nukleinsäuren auf; und/oder
b) die nukleinsäurebindenden Gruppen sind ausgewählt aus der Gruppe der Mono- und Diamine; und/oder
c) der Träger ist mit einer Mischung aus nukleinsäurebindenden Gruppen und Verdünnungsgruppen beschichtet, wie in Anspruch 1 definiert.

Offenbart wird, dass die nukleinsäurebindenden Liganden im Unterschuss an den Träger gebunden vorliegen können.

Die nukleinsäurebindende Phase, die erfindungsgemäß zum Einsatz kommt, weist insbesondere nukleinsäurebindende Liganden mit wenigstens einer protonierbaren Gruppe auf, die einen pKs-Wert von wenigstens 8, vorzugsweise 9 bis 12 aufweist. Bevorzugte Ausgestaltungen sind oben im Detail beschrieben (siehe obige Offenbarung) und insbesondere durch ein oder mehrere der folgenden Merkmale gekennzeichnet:
a. die protonierbaren Gruppe(n) weisen einen pKs-Wert von 9, bis 12 vorzugsweise einen pKs-Wert von 10 bis 12 auf; und/oder
b. die protonierbaren Gruppen sind Aminogruppen und liegen nicht in Konjugation mit die Elektronendichte verringernden Gruppen vor; und/oder
c. die nukleinsäurebindende Phase weist funktionelle Gruppen auf, die bei dem Elutions pH-Wert die Freisetzung/Elution der Nukleinsäuren begünstigen, vorzugsweise Kationenaustauscher, insbesondere Carboxylgruppen; und/oder
d. der Träger weist keine eigenen nukleinsäurebindenden Eigenschaften neben den nukleinsäurebindenden Liganden auf; und/oder
e. der Träger ist ausgewählt aus der Gruppe bestehend aus organischen Polymeren wie Polystyrol und seinen Derivaten, Polyacrylaten und - methacrylaten und ihren Derivaten, Polyurethanen, Nylon, Polyethylen, Polypropylen, Polybutylen und Copolymeren aus diesen Materialien, Polysacchariden und Hydrogelen wie Agarose, Cellulose, Dextran, Sephadex, Sephacryl, Chitosan, anorganischen Trägern, Glas oder weiteren Metall- und Halbmetalloxiden (insbesondere Oxide der Formel MeO, wobei Me vorzugsweise ausgewählt ist aus der Gruppe umfassend Al, Ti, Zr, Si, insbesondere Al₂O₃, TiO₂, Silica), Trägern mit Metalloberflächen, wie z. B. Gold, magnetische Partikel oder Mischungen derselben; und/oder
f. die nukleinsäurebindenden Liganden sind ausgewählt aus der Gruppe bestehend aus primären, sekundären und tertiären Aminen der Formeln
   (a) R₃N
   (b) R₂NH
   (c) RNH₂ und/oder
   (d) X-(CH₂)ₙ-Y mit X = R₂N, RNH oder NH₂ und Y = R₂N oder RNH oder NH₂
   wobei
   R = unabhängig voneinander ein verzweigter, unverzweigter oder ringförmiger Alkyl-, Alkenyl-, Alkinyl- oder aromatischer Substituent ist, wobei dieser auch ein oder mehrere Heteroatome enthalten kann;
   n= 0 bis 20,
   und stellen vorzugsweise Mono- und Diamine dar. Diese können substituiert oder unsubstituiert sein.

Ferner wird ein Kit zur Aufreinigung einer definierten Menge von Nukleinsäuren zur Verfügung gestellt, bei dem eine erfindungsgemäße nukleinsäurebindende Phase eingesetzt wird. Einzelheiten zu der nukleinsäurebindenden Phase und den Vorteilen in Bezug auf die Normalisierung sind oben im Detail beschrieben und gelten auch im Zusammenhang mit dem Kit. Wir verweisen auf die obige Offenbarung.

Das Kit weist vorzugsweise wenigstens eines der folgenden Merkmale auf:
a. einen Bindungspuffer bzw. eine Bindungslösung mit einem pH-Wert, der wenigstens eine pH Einheit unterhalb des pKs-Wertes wenigstens einer der protonierbaren Gruppen der nukleinsäurebindenden Phase liegt, und/oder einen Bindungspuffer, der die Einstellung eines solchen pH-Wertes in der Probe ermöglicht; und/oder
b. einen Elutionspuffer bzw. eine Elutionslösung mit einem pH-Wert, der eine pH Einheit unterhalb des pKs-Wertes wenigstens einer der protonierbaren Gruppen der nukleinsäurebindenden Phase liegt, und/oder einen Elutionspuffer, der die Einstellung eines solchen pH-Wertes in der Probe ermöglicht.

Gemäß einer Ausführungsform weist das Kit einen Bindungspuffer bzw. Bindungslösung auf, der wenigstens eines der folgenden Merkmale aufweist:
i. einen pH-Wert von 3 bis 8; und/oder
ii. einen pH-Wert von 4 bis 7,5; und/oder
iii. einen pH-Wert von 4,5 bis 7; und/oder
iv. einen pH-Wert von 5,5 bis 7; und/oder
v. einen pH-Wert von 6,5 bis 7; und/oder
vi. eine Salzkonzentration von ≤ 1M, ≤ 0,5M, ≤ 0,25M oder w≤ 0,1M;

Das Kit kann ferner einen Elutionspuffer bzw. Elutionslösung aufweisen, der wenigstens eines der folgenden Merkmale aufweist:
i. einen pH-Wert von 7,5 bis 10; und/oder
ii. einen pH-Wert von 8 bis 9; und/oder
iii. einen pH-Wert von 8,2 bis 8,8; und/oder
iv. eine Salzkonzentration von ≤ 1M, ≤ 0,5M, ≤ 0,25M, ≤ 0,1 M, ≤ 25mM, ≤ 15mM oder ≤ 10mM; und/oder
v. er ist ausgewählt aus der Gruppe bestehend aus Wasser, biologischem und organischem Puffer.

Einzelheiten zur nukleinsäurebindenden Phase sowie den Bindungs- und Elutionsbedingungen sind im Detail oben beschrieben und gelten auch im Zusammenhang mit dem Kit und kennzeichnen die darin verwendeten Bestandteile/Puffer. Wir verweisen auf die obige Offenbarung. Darüber hinaus kann das Kit weitere übliche Bestandteile enthalten, so bspw. Lyse-, Wasch - und/oder Neutralisierungsreagenzien bzw. Puffer.

Die entsprechenden Kits können insbesondere im Rahmen des erfindungsgemäßen Verfahrens zur Anwendung kommen und sind besonders zur Normalisierung geeignet. Daher kann das Kit insbesondere bei automatisierten Systemen zum Einsatz kommen.

Die vorliegenden Verfahren, Kits sowie nukleinsäurebindenden festen Phasen können insbesondere im Bereich der Molekularbiologie, der molekularen Diagnostik, in der Forensik, in der Lebensmittelanalytik sowie im Applied Testing eingesetzt werden. Sie sind insbesondere für die automatisierte Anwendung geeignet.

Die eluierten Nukleinsäuren können vorzugsweise direkt weiterverarbeitet werden, so bspw. im Rahmen molekularbiologischer Anwendungen wie bspw. enzymatischen Reaktionen wie bspw. einer PCR, RT-PCR, einem Restriktionsverdau, einer Sequenzierung oder einer Transkription zum Einsatz kommen. Eine weitere Aufreinigung ist nicht erforderlich, sofern die Elutionspuffer wie oben beschrieben ausgestaltet sind und vorzugsweise eine niedrige Salzkonzentration aufweisen.

Als Nukleinsäuren kommen für eine Aufreinigung DNA und RNA in Frage, insbesondere genomische DNA, Plasmid-DNA, sowie PCR Fragmente, cDNA, miRNA, siRNA, sowie Oligonukleotide und modifizierte Nukleinsäuren wie bspw. PMA oder LMA. Es können auch virale oder bakterielle RNA sowie DNA oder Nukleinsäuren aus menschlichen, tierischen oder pflanzlichen Quellen aufgereinigt werden. Des Weiteren kommen für eine erfindungsgemäße Aufreinigung auch DNA/RNA Hybride in Frage, um nur einige Beispiele zu nennen.

Die vorliegende Erfindung soll nachfolgend anhand von einigen Beispielen erläutert werden. Diese sind nicht beschränkend, stellen jedoch bevorzugte Ausführungsformen der vorliegenden Erfindung dar.

### BEISPIELE

Im Rahmen der Experimente wurden als Modellsysteme Long Range (LR) PCR Fragmente (8 bis 11 kb) eingesetzt. Die Versuche wurden anhand der nachfolgenden Vorschriften durchgeführt.

**Fig. 1** zeigt die Abhängigkeit der eluierten DNA-Menge (LR PCR Fragmente) von der Ausgangs-DNA Menge bei Einsatz von mit Spermin beschichteten Polymerbeads. Je mehr DNA den mit Polyaminen beschichteten Partikeln angeboten wurden, desto mehr DNA konnte gebunden und dann wieder eluiert werden. Ein Plateau der Nukleinsäurebindekapazität wird jedoch selbst bei größeren Nukleinsäuremengen in der Probe nicht erreicht, eine Normalisierung trat auch bei Einsatz verschiedener Ausgangs-DNA Mengen nicht ein.

### A) Umsetzung von magnetischen Polymeren mit Aminen

### Materialien

Als magnetisches Polymer können verschiedene Materialien eingesetzt werden, darunter Estapor, Dynal, Seradyn oder Ademtech Partikel.

### Amin: N-Propyl-1,3-propandiamin (Aldrich, Katalog-Nr. 308153)

### Herstellvorschrift

Man resuspendiert 500 mg carboxylierte Magnetpartikel in 10 ml MES Puffer und setzt dann 10 ml einer 50 mg/ml Lösung von N-Hydroxysuccinimid zu. Man mischt mit einem Minishaker, setzt dann 10 ml einer 50 µmol/l Lösung von 1-Ethyl-3-(3-dimethylaminopropyl)carbodiimid zu und vortext erneut. Anschließend lässt man 30 Minuten am Überkopfschüttler reagieren und trennt dann den Überstand ab. Man resuspendiert in 50 ml MES Puffer. Die Suspension wird magnetisch separiert und der Überstand verworfen. Dann nimmt man in 5 ml MES Puffer auf und setzt 10 ml einer wässrigen 1,4-Diaminobutan Lösung zu (100 mg/ml in MES, pH-Wert 8,5), vortext gründlich und lässt eine Stunde am Überkopfschüttler reagieren. Anschließend wäscht man zweimal mit je 50 ml MES Puffer, separiert magnetisch und verwirft die Überstände. Die Partikel werden dann in 10 ml MES-Puffer resuspendiert.

### B) Normalisierung von LR-PCR Fragmenten

### 1) Normalisierung unterschiedlicher Ausgangs-DNA-Mengen derselben Probe

### Waschen der Beads:

4 Ansätze: je 2µl Beads mit 2x100µl 50mM TrisHCl pH6,5 waschen

Die Konzentrierung der Beads erfolgt jeweils für mindestens 30 sec an einem Magneten, bevor der Überstand abgenommen wird.

### Bindung der DNA:

Suspendieren der Beads in 100µl Waschpuffer (s.o.) + 2µl 3M NaAc pH5,3
**1** + 4µl DNA LR-PCR06 (8 kb Fragment)
**2** + 3µl DNA LR-PCR06 + 1µl LR-PCR-Puffer 1 x
**3** + 2µl DNA LR-PCR06 + 2µl LR-PCR-Puffer 1x
**4** + 1µl DNA LR-PCR06 + 3µl LR-PCR-Puffer 1x

### 10min bei RT schütteln

### Waschen:

2x Waschen mit 100µl Wasser

### Elution:

+ 20µl 50mM Tris-HCl pH 8,5, 50mM NaCl
5 min RT

Überführen des Überstand in neue Gefäße.

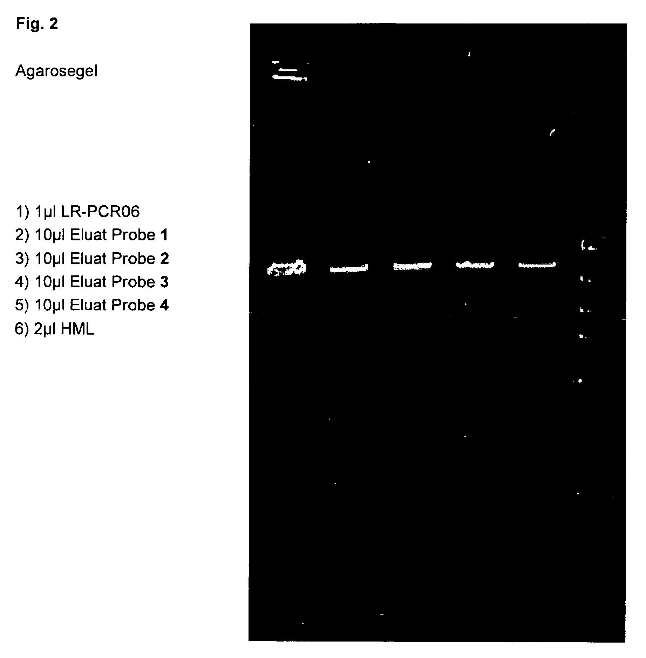

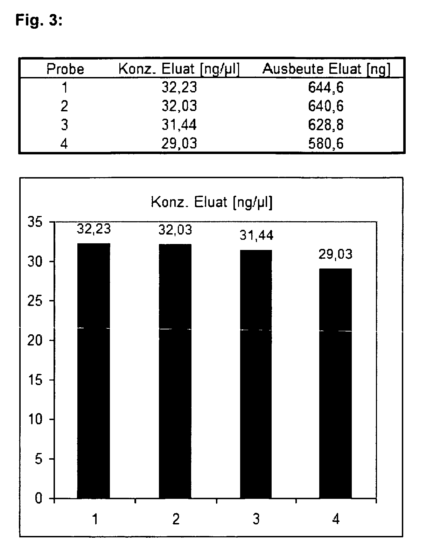

Die Ergebnisse der Normalisierung sind in den **Fig. 2** und **3** zusammengefasst.

### 2) Normalisierung unterschiedlicher DNA-Fragmente zwischen 8 und 11 kb

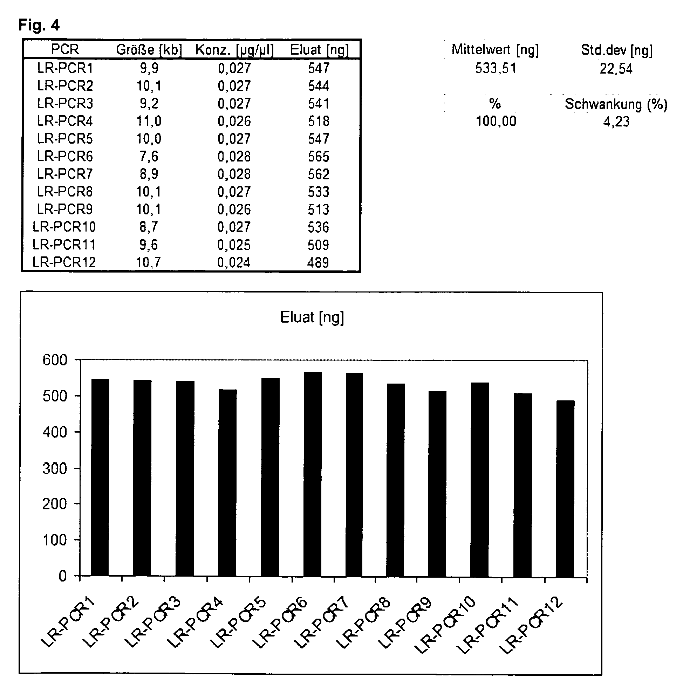

12 unterschiedliche Long Range PCR-Proben wurden nach dem Protokoll gemäß 1) aufgereinigt und normalisiert. **Fig. 4** fasst die DNA-Mengen im Eluat nach Pico-Green-Messung zusammen.

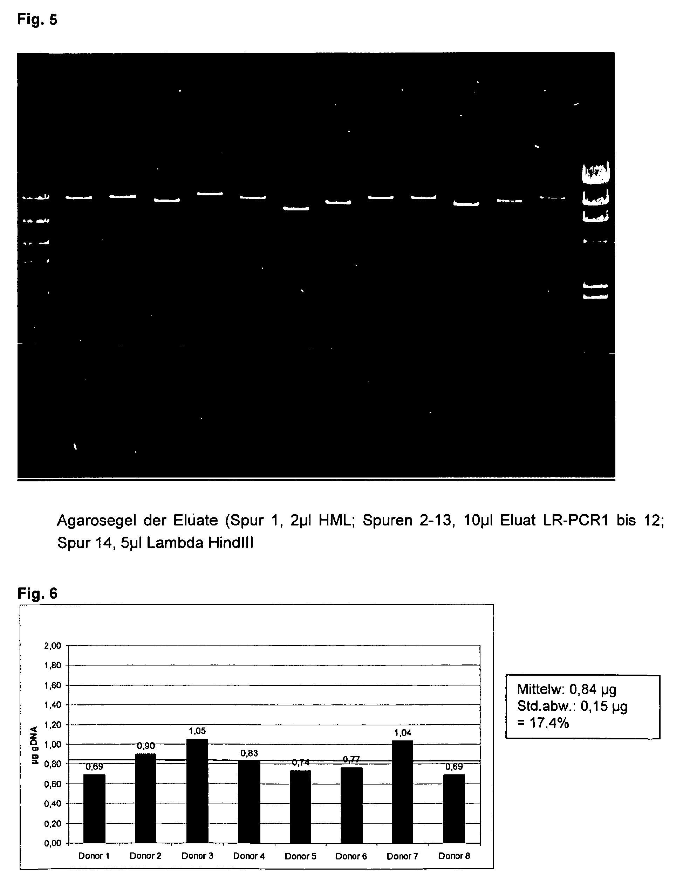

Anschließend wurden die 12 Proben zusammengefasst und die durchschnittliche Konzentration durch OD₂₆₀ₙₘ Messung kontrolliert. Hier wurde ein durchschnittlicher Wert von 580ng pro PCR-Fragment bestimmt, was mit dem Durchschnittswert nach PicoGreen-Messung von 533ng vergleichbar ist. **Fig. 5** zeigt die Ergebnisse. Die schwächeren Banden in den Spuren 12 und 13 lassen sich durch einen erhöhten DNA-Hintergrund, der durch unspezifische Amplifikation verursacht wurde, erklären.

In verschiedenen Versuchen, konnte gezeigt werden, dass Fragmente ähnlicher Größe (8-11kb) und unterschiedlicher Größe (z.B. 1kb, 5kb, 10kb; Ergebnisse nicht dargestellt) mit geringen Schwankungen unter 10% normalisiert werden konnten. Damit stellt das hier vorgestellte Verfahren eine deutliche Verbesserung gegenüber den bisher bekannten Verfahren der Normalisierung dar, deren Schwankungen in den Ausbeuten von 50% bzw. 2-3fach deutlich größer sind.

Wenn das vorliegende Verfahren zur Normalisierung von PCR-Fragmenten eingesetzt wird, so ist durch die Kenntnis der Fragmentlängen neben der Normalisierung der DNA-Massen auch eine Normalisierung der Fragmentmengen (Molarität) mittels der Formel

### Volume[µl]=MW[µg/µmol]/conc[µg/µl]

### möglich.

Das erfindungsgemäße Verfahren hat daher erhebliche Vorteile gegenüber dem Stand der Technik. So wird eine sehr gute Ausbeute von 500 ng pro 2 µl Beads in 20 µl Eluat erreicht, wobei Schwankungen von lediglich +/- 10 % auftreten. Das erfindungsgemäße Verfahren kann automatisiert und mit magnetischen Separatoren, Pipetten und Shakern eingesetzt werden. Darüber hinaus ist es besonders zeiteffizient, da innerhalb von 20 Minuten 96 Proben in einem automatisierten Verfahren aufgereinigt werden können. Dies ist ein erheblicher Vorteil gegenüber dem Stand der Technik, wo teilweise 60 Minuten und mehr für die Aufreinigung der gleichen Probenmenge benötigt werden.

### C) Normalisierung von gDNA aus Blut

### 1) Isolierung von gDNA aus Blut von acht verschiedenen Spendern

### Materialien

Probe: Frisches Vollblut von 8 verschiedenen Spendern, EDTA stabilisiert.
Beads: 1 mg N-Propyl-1,3-propandiamin beschichtet magnetische Beads (siehe oben) Lysepuffer: 10 mM TRIS, Triton X-100, pH 9,0
Bindepuffer: 1,5 M Kaliumacetat pH 4,0
Waschpuffer = Wasser
Elutionspuffer: 10 mM TRIS*HCl pH 8,5

### Durchführung:

Die Aufreinigung erfolgte nach folgender Versuchsanleitung:

1 ml Lysepuffer und 10 µl Proteinase K werden gemischt. 1 mg Beads und 3,4 µl Wasser werden in 200 µl Bindpuffer suspendiert. 100 µl Blut werden in einem Eppendorf Gefäß mit dem Lysepuffer/Proteinase Mix versetzt und gut gemischt. Es wird bei Raumtemperatur 10 min. inkubiert. Die Beads werden im Bindepuffer sorgfältig resuspendiert. Davon werden 240 µl zu dem lysierten Blut gegeben. Es wird sorgfältig gemischt durch auf- und abpipettieren. Bei Raumtemperatur wird 1 min. inkubiert. Die Beads werden magnetisch separiert. Der Überstand wird vorsichtig abgenommen. Zum Waschen wird 1 ml Waschpuffer zugeben. Es wird sorgfältig gemischt durch auf- und abpipettieren. Es wird erneut magnetisch separiert und der Überstand verworfen. Es wird 1 ml Lysepuffer und 50 µl Bindepuffer zugegeben, sorgfältig gemischt und eine Minute bei RT inkubiert. Es wird magnetisch separiert. Anschließend wird ein weiteres Mal mit 1 ml Waschpuffer gewaschen und magnetisch separiert.

Um die aufgereinigte DNA zu eluieren werden die Beads mit 150 µl Elutionspuffer versetzt. Die Beads werden durch mehrmaliges Auf- und Abpipettieren resuspendiert. Die Beads werden magnetisch separiert, der Überstand wird abgenommen und die DNA photometrisch quantifiziert.

### Ergebnis:

Die Ergebnisse sind in Fig. 6 gezeigt. Die Daten zeigen, dass trotzdem die eingesetzten Ausgangsmaterialien sehr komplex sind und unterschiedliche Mengen an Nukleinsäuren enthalten können, eine normlaisierte DNA Menge nach dem erfindungsgemäßen Verfahren aufgereinigt werden konnte, die nur geringeren Schwankungen unterlag.

### 2) Isolierung unter bewusster Variation der DNA Ausgangsmenge

Um zu prüfen, ob das erfindungsgemäße Verfahren eine DNA-Normalisierung auch bei komplexen Proben erzielt, die stark unterschiedliche DNA Mengen enthalten, wurde der nachfolgende Versuch durchgeführt.

### Materialien

Probe: Frisches Vollblut, EDTA stabilisiert.
Beads: 600 µg N-Propyl-1,3-propandiamin beschichtete magnetische Beads
Lysepuffer: 10 mM TRIS, Triton X-100, pH 9,0
Bindepuffer: 1,5 M Kaliumacetat pH 4,0
Waschpuffer = Wasser
Elutionspuffer: 10 mM TRIS*HCl pH 8,5

Die theoretische Ausgangsmenge der gDNA wurde durch Auszählen der weißen Blutkörperchen bestimmt (WBC Count = White Blood Cell Count). Dabei wurden 6,6 pg DNA pro Zelle vorausgesetzt.

| **Blutvolumen** | **WBC** | **DNA-Menge** |
|---|---|---|
| | x10^3/µl | µg DNA |
| 50µl | 4,7 | 1,55 |
| 100µl | 4,7 | 3,10 |
| 150µl | 4,7 | 4,65 |
| 200µl | 4,7 | 6,20 |
| 250µl | 4,7 | 7,76 |
| 300µl | 4,7 | 9,31 |

Zusammensetzung des Lysemix:

| **Blutvolumen** | **Proteinase K [µl]** | **Lysepuffer [ml]** |
|---|---|---|
| 50µl | 10 | 1 |
| 100µl | 10 | 1 |
| 150µl | 15 | 1 |
| 200µl | 20 | 1 |
| 250µl | 25 | 1 |
| 300µl | 30 | 1 |

### Durchführung:

Die Aufreinigung erfolgte nach folgender Versuchsanleitung:

Die oben angegebenen Mengen an Lysepuffer und Proteinase K werden jeweils gemischt. 600 µg Beads (= 16,9 µl) und 23,1 µl Wasser werden jeweils in 200 µl Bindepuffer suspendiert. Die oben angegebene Menge Blut wird jeweils in einem Eppendorfgefäß mit dem Lysepuffer/Proteinase Mix versetzt und gut gemischt. Es wird bei RT 10 min. inkubiert. Die Beads werden im Bindepuffer sorgfältig resuspendiert. Davon werden 240 µl zu dem lysierten Blut gegeben. Durch auf- und abpipettieren wird sorgfältig gemischt. Bei Raumtemperatur wird 1 min. inkubiert. Die Beads werden magnetisch separiert. Der Überstand wird vorsichtig abgenommen. Zum Waschen wird 1 ml Waschpuffer zugeben. Durch auf- und abpipettieren wird sorgfältig gemischt. Es wird erneut magnetisch separiert und der Überstand verworfen. Es wird 1 ml Lysepuffer und 50 µl Bindepuffer zugegeben, sorgfältig gemischt und eine Minute bei RT inkubiert. Es wird magnetisch separiert. Anschließend wird ein weiteres Mal mit 1 ml Waschpuffer gewaschen und magnetisch separiert.

Um die aufgereinigte DNA zu eluieren, werden die Beads mit 150 µl Elutionspuffer versetzt. Die Beads werden durch mehrmaliges Auf- und Abpipettieren resuspendiert. Die Beads werden magnetisch separiert, der Überstand wird abgenommen und die DNA photometrisch quantifiziert.

### Ergebnis:

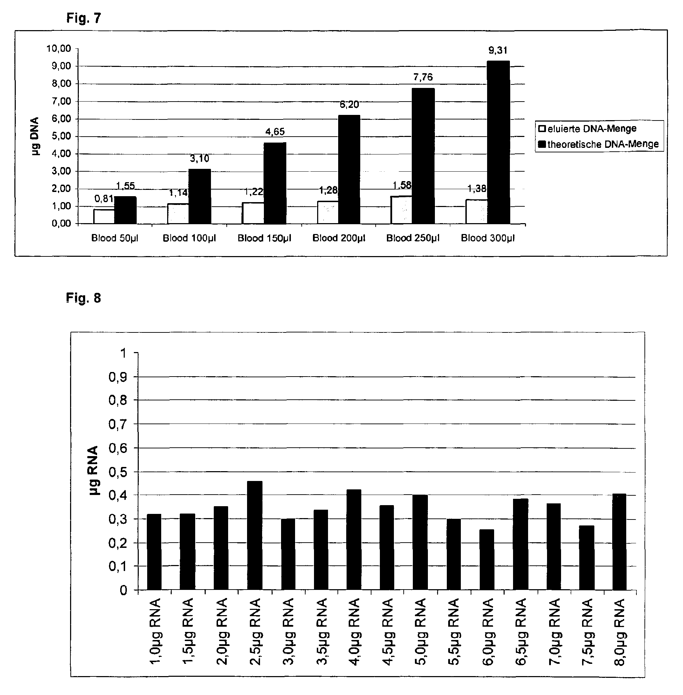

Die Ergebnisse sind in Fig. 7 und in der nachfolgenden Tabelle gezeigt. Die Daten zeigen, dass trotzdem die eingesetzten Ausgangsmaterialien sehr unterschiedliche Mengen an Nukleinsäuren enthielten, eine normalisierte DNA Menge nach dem erfindungsgemäßen Verfahren aufgereinigt werden konnte, die erheblich geringeren Schwankungen unterlag als das Ausgangsmaterial.

| Mittelw | Stdabw | % |
|---|---|---|
| Eingangs DNA: | | |
| WBC | | |
| **5,43** | **2,90** | **53,45** |
| Ausgangs DNA: | | |
| AX Beads | | |
| **1,24** | **0,26** | **20,76** |

### D.) Normalisierung von RNA

### Materialien:

Probe: RNA Boehringer Mannheim 16s-and 23s- ribosomal β=4µg RNA/µl
Beads: 38 µg N-Propyl-1,3-propandiamin beschichtete magnetische Beads
Beadwaschpuffer: 50 mM TRIS*HCl pH6,5 (RNasefrei)
Bindepuffer: 3 M Natriumacetat pH5,3 (RNasefrei)
Waschpuffer = Wasser (RNasefrei)
Elutionspuffer: 50 mM TRIS*HCl/50 mM NaCl pH8,5 (RNasefrei)
RNA-Zugabemengen: 1 - 8 µg (0,5µg Schritte)
RNA-Zugabevolumen: 4 µl (Volumendifferenzen mit RNasefreiem Wasser angeglichen)

### Durchführung:

Die Aufreinigung erfolgte nach folgender Versuchsanleitung:
Die Beads werden sorgfältig resuspendiert und 38 µg in eine Microtitierplatte gegeben. Es wird magnetisiert, der Puffer abgenommen und die Beads in 100 µl Beadwaschpuffer aufgenommen. Dieser Schritt wird noch einmal wiederholt. Dann fügt man 2 µl Bindepuffer und 4 µl der verschiedenen RNA-Lösungen hinzu. Es wird sorgfältig gemischt durch auf- und abpipettieren. Die Platte wird für 10 Minuten bei 1000 rpm und Raumtemperatur (RT) geschüttelt. Die Beads werden magnetisch separiert. Der Überstand wird vorsichtig abgenommen.

Zum Waschen werden 100 µl Waschpuffer zugeben. Es wird sorgfältig gemischt durch auf- und abpipettieren. Es wird erneut magnetisch separiert und der Überstand verworfen. Der Waschschritt wir noch einmal wiederholt.

Um die RNA zu eluieren werden die Beads mit 20 µl Elutionspuffer versetzt. Die Beads werden durch mehrmaliges Auf- und Abpipettieren resuspendiert und 5 Minuten bei 1000 rpm und RT geschüttelt. Die Beads werden magnetisch separiert, der Überstand wird abgenommen und die RNA photometrisch quantifiziert.

### Ergebnis:

Die Ergebnisse sind in Fig. 8 und in der nachfolgenden Tabelle gezeigt. Die Daten zeigen, dass trotzdem die eingesetzten Ausgangsmaterialien sehr unterschiedliche Mengen an Nukleinsäuren enthielten, eine normalisierte RNA Menge nach dem erfindungsgemäßen Verfahren aufgereinigt werden konnte, die erheblich geringeren Schwankungen unterlag als das Ausgangsmaterial.

| Mittelw | Stdabw | % |
|---|---|---|
| Eingangs RNA 1,0 - 8,0µg RNA | | |
| 4,5 | 2,236 | 50% |
| Ausgangs RNA: | | |
| 0,35 | 0,057 | 16% |

## Patentansprüche

1. Verfahren zur Isolierung und/oder Aufreinigung einer definierten Menge von Nukleinsäuren aus einer nukleinsäurehaltigen Probe, das wenigstens die nachfolgenden Schritte aufweist:
a. Inkontaktbringen der nukleinsäurehaltigen Probe mit einer definierten Menge einer nukleinsäurebindenden Phase mit folgenden Merkmalen:
(i) die nukleinsäurebindende Phase weist nukleinsäurebindende Liganden auf, die wenigstens eine protonierbare Gruppe aufweisen;
(ii) die nukleinsäurebindenden Liganden liegen an einen Träger gebunden vor;
(iii) die nukleinsäurebindende Phase weist eine Oberfläche mit einer niedrigen Ladungsdichte auf, wobei die niedrige Ladungsdichte durch eines oder mehrere der nachfolgenden Merkmale erzielt wird:
aa) die nukleinsäurebindenden Liganden weisen am Träger gebunden jeweils nicht mehr als ein oder zwei protonierbare Gruppen zur Bindung der Nukleinsäuren auf; und/oder
bb) die nukleinsäurebindenden Liganden sind ausgewählt aus der Gruppe der Mono- und Diamine; und/oder
cc) der Träger ist mit einer Mischung aus nukleinsäurebindenden Liganden und Verdünnungsliganden beschichtet, wobei die Verdünnungsliganden keine Nukleinsäuren binden oder die Nukleinsäuren mit einer geringeren Affinität als die nukleinsäurebindenen Liganden binden, und wobei der Anteil der nukleinsäurebindenen Liganden im Verhältnis zu den Verdünnungsliganden ≤ 50% beträgt,
wobei die Menge an Nukleinsäuren in der Probe die Bindungskapazität der eingesetzten Menge an nukleinsäurebindender Phase übersteigt;
b. Bindung der Nukleinsäuren an die nukleinsäurebindende Phase bei einem pH-Wert (Bindungs pH-Wert), der unterhalb des pKs-Wertes wenigstens einer der protonierbaren Gruppen liegt;
c. Elution der Nukleinsäuren bei einem pH-Wert, der oberhalb des Bindungs pH-Wertes liegt, wobei eine definierte Menge an Nukleinsäuren erhalten wird.

2. Das Verfahren nach Anspruch 1, **dadurch gekennzeichnet, dass** gemäß cc) der Anteil der nukleinsäurebindenen Liganden im Verhältnis zu den Verdünnungsliganden ≤ 25%, ≤ 15%, ≤ 10% oder nur ≤ 5% beträgt.

3. Das Verfahren nach Anspruch 1 oder 2, **dadurch gekennzeichnet, dass** die protonierbaren Gruppe(n) eines oder mehrere der nachfolgenden Merkmale aufweisen:
a. einen pKs-Wert von 9 bis 12;
b. einen pKs-Wert von 10 bis 12;
c. die protonierbaren Gruppen sind Aminogruppen und liegen nicht in Konjugation mit die Elektronendichte verringernden Gruppen vor.

4. Das Verfahren nach einem oder mehreren der Ansprüche 1 bis 3, **dadurch gekennzeichnet, dass** die nukleinsäurebindende Phase wenigstens eines oder mehrere der nachfolgenden Merkmale aufweist:
a. der Träger und/oder die nukleinsäurebindenden Liganden weisen funktionelle Gruppen auf, die beim Elutions pH-Wert die Freisetzung der Nukleinsäuren fördern, nämlich Kationenaustauscher als funktionelle Gruppen, insbesondere saure Gruppen wie vorzugsweise Carboxylgruppen;
b. die nukleinsäurebindenden Liganden sind ausgewählt aus der Gruppe bestehend aus primären, sekundären und tertiären Aminen der Formeln
R₃N,
R₂NH,
RNH₂
und/oder
X-(CH₂)ₙ-Y
mit
X = R₂N, RNH oder NH₂
Y = R₂N oder RNH oder NH₂
R = unabhängig voneinander ein verzweigter, unverzweigter oder ringförmiger Alkyl-, Alkenyl-, Alkinyl- oder aromatischer Substituent, wobei dieser auch ein oder mehrere Heteroatome enthalten kann;
n= 0 bis 20, vorzugsweise 0 bis 18;
c. der Träger weist keine eigenen nukleinsäurebindenden Eigenschaften neben den nukleinsäurebindenden Liganden auf;
d. der Träger ist ausgewählt aus der Gruppe bestehend aus organische Polymere wie Polystyrol und seine Derivate, Polyacrylate und -methacrylate und ihre Derivate, Polyurethane, Nylon, Polyethylen, Polypropylen, Polybutylen und Copolymere aus diesen Materialien, Polysaccharide und Hydrogele wie Agarose, Cellulose, Dextran, Sephadex, Sephacryl, Chitosan, anorganische Träger, Glas, Metall- und Halbmetalloxide, insbesondere Al₂O₃, TiO₂, Silica, Boroxid, Träger mit Metalloberflächen, wie z. B. Gold und magnetische Partikel und Mischungen davon, Röhrchen, Membrane, Vliese, Papier, Reaktionsgefäße, Multiplates, Chips und Microarrays; und/oder
e. die Bindekapazität liegt in einem Bereich von 0,1 bis 500µg Nukleinsäure pro mg nukleinsäurebindender Phase, vorzugsweise bevorzugt 0,1 bis 10µg Nukleinsäure pro mg nukleinsäurebindender Phase; und/oder
f. die Bindekapazität liegt in einem Bereich von 0,01 bis 10µg, 0,025 bis 10µg, 0,05 bis 10µg oder 0,1 bis 10µg Nukleinsäure pro mg nukleinsäurebindender Phase

5. Das Verfahren nach einem oder mehreren der Ansprüche 1 bis 4, **dadurch gekennzeichnet, dass** die Bindung unter Bedingungen erfolgt, die wenigstens eines der nachfolgenden Merkmale aufweisen:
a. die Bindung erfolgt bei einem pH-Wert von 3 bis 8; und/oder
b. die Bindung erfolgt bei einem pH-Wert von 4 bis 7,5; und/oder
c. die Bindung erfolgt bei einem pH-Wert von 4,5 bis 7; und/oder
d. die Bindung erfolgt bei einem pH-Wert von 5,5 bis 7; und/oder
e. die Bindung erfolgt bei einem pH-Wert von 6,5 bis 7; und/oder
f. die Bindung erfolgt bei einer Salzkonzentration von ≤ 1M, ≤ 0,5M, ≤ 0,25M oder ≤ 0,1 M.

6. Das Verfahren nach einem oder mehreren der Ansprüche 1 bis 5, **dadurch gekennzeichnet, dass** die Elution unter Bedingungen erfolgt, die wenigstens eines der nachfolgenden Merkmale aufweisen:
a. die Elution erfolgt bei einem pH-Wert, der oberhalb des Bindungs pH-Wertes liegt, jedoch wenigstens eine pH-Einheit unterhalb des pKs-Wertes wenigstens einer der protonierbaren Gruppen liegt; und/oder
b. die Elution erfolgt bei einem pH-Wert von 7,5 bis 10; und/oder
c. die Elution erfolgt bei einem pH-Wert von 8 bis 9; und/oder
d. die Elution erfolgt bei einem pH-Wert von 8,2 bis 8,8; und/oder
e. die Elution erfolgt bei einer Salzkonzentration von ≤ 1M, ≤ 0,5M, ≤ 0,25M, ≤ 0,1M, ≤ 25mM, ≤ 15mM oder ≤ 10mM; und/oder
f. zur Elution wird eine Lösung eingesetzt, die ausgewählt ist aus der Gruppe von Wasser, biologischem und organischem Puffer.

7. Das Verfahren nach einem oder mehreren der Ansprüche 1 bis 6, **dadurch gekennzeichnet, dass** das Verfahren im Anschluss an ein Nukleinsäureaufreinigungsverfahren durchgeführt wird, um bereits aufgereinigte Nukleinsäuren zu normalisieren und so eine definierte Menge an Nukleinsäuren zu erhalten.

8. Das Verfahren nach einem oder mehreren der Ansprüche 1 bis 7, **dadurch gekennzeichnet, dass** die normalisierte Nukleinsäure für eine enzymatische Reaktion eingesetzt wird, vorzugsweise in einer Nukleinsäureamplifikation, Modifikationsreaktion oder Sequenzierreaktion.

9. Das Verfahren nach einem oder mehreren der Ansprüche 1 bis 8, **dadurch gekennzeichnet, dass** es sich bei der Nukleinsäure um PCR-Fragmente handelt.

10. Das Verfahren nach einem oder mehreren der vorherigen Ansprüche, **dadurch gekennzeichnet, dass** der Träger durch nichtmagnetische oder magnetische Partikel bereitgestellt wird.

11. Das Verfahren nach einem oder mehreren der vorherigen Ansprüche, **dadurch gekennzeichnet, dass** der nukleinsäurebindende Ligand N-Propyl-1,3 Propandiamin ist.

12. Verwendung einer nukleinsäurebindenden Phase zur Isolierung und/oder Aufreinigung einer definierten Menge an Nukleinsäuren aus einer nukleinsäurehaltigen Probe, **dadurch gekennzeichnet, dass** die nukleinsäurebindende Phase nukleinsäurebindende Liganden mit wenigstens einer protonierbaren Gruppe aufweist, wobei die nukleinsäurebindenden Liganden an einen Träger gebunden vorliegen und die nukleinsäurebindende Phase eine Oberfläche mit einer niedrigen Ladungsdichte aufweist, wobei die niedrige Ladungsdichte durch ein oder mehrere der nachfolgenden Merkmale erzielt wird:
a. die nukleinsäurebindenden Liganden weisen am Träger gebunden jeweils nicht mehr als ein oder zwei protonierbare Gruppen zur Bindung der Nukleinsäuren auf; und/oder
b. die nukleinsäurebindenden Liganden sind ausgewählt aus der Gruppe der Mono- und Diamine; und/oder
c. der Träger ist mit einer Mischung aus nukleinsäurebindenden Liganden und Verdünnungsliganden beschichtet, wobei die Verdünnungsliganden keine Nukleinsäuren binden oder die Nukleinsäuren mit einer geringeren Affinität als die nukleinsäurebindenen Liganden binden und wobei der Anteil der nukleinsäurebindenen Liganden im Verhältnis zu den Verdünnungsliganden ≤ 50% beträgt;
wobei ein Elutions pH-Wert eingestellt wird, der oberhalb des Bindungs pH-Wertes liegt und wobei die Menge an nukleinsäurebindender Phase so gewählt ist, dass die Nukleinsäuren in der Probe im Überschuss zu der Bindungskapazität der eingesetzten nukleinsäurebindenden Phase vorliegen.

13. Verwendung nach Anspruch 12, **dadurch gekennzeichnet, dass** die nukleinsäurebindende Phase wenigstens eines der nachfolgenden Merkmale aufweist:
a. die protonierbaren Gruppe(n) weisen einen pKs-Wert von 9, bis 12 vorzugsweise einen pKs-Wert von 10 bis 12 auf; und/oder
b. die protonierbaren Gruppen sind Aminogruppen und liegen nicht in Konjugation mit die Elektronendichte verringernden Gruppen vor; und/oder
c. die nukleinsäurebindende Phase weist funktionelle Gruppen auf, die bei dem Elutions pH-Wert die Freisetzung/Elution der Nukleinsäuren begünstigt, vorzugsweise Kationenaustauscher, insbesondere Carboxylgruppen; und/oder
d. die Bindekapazität der nukleinsäurebindenden Phase liegt in einem Bereich von 0,1 bis 500µg Nukleinsäure pro mg nukleinsäurebindender Phase, vorzugsweise 0,1 bis 200µg, besonders bevorzugt 0,1 bis 10µg Nukleinsäure pro mg nukleinsäurebindender Phase;
e. der Träger weist keine eigenen nukleinsäurebindenden Eigenschaften neben den nukleinsäurebindenden Liganden auf; und/oder
f. der Träger ist ausgewählt aus der Gruppe bestehend aus organischen Polymeren wie Polystyrol und seinen Derivaten, Polyacrylaten und - methacrylaten und ihren Derivaten, Polyurethanen, Nylon, Polyethylen, Polypropylen, Polybutylen und Copolymeren aus diesen Materialien, Polysacchariden und Hydrogelen wie Agarose, Cellulose, Dextran, Sephadex, Sephacryl, Chitosan, anorganischen Trägern, Glas oder weiteren Metall- und Halbmetalloxiden, insbesondere Al₂O₃, TiO₂, Silica, Boroxid, Trägern mit Metalloberflächen, wie z. B. Gold, magnetischen Partikeln oder Mischungen derselben, Röhrchen, Membrane, Vliese, Papier, Reaktionsgefäße, Multiplates, Chips und Microarrays; und/oder
g. die nukleinsäurebindenden Liganden sind ausgewählt aus der Gruppe bestehend aus primären, sekundären und tertiären Aminen der Formeln
R₃N, R₂NH, RNH₂ und/oder X-(CH₂)ₙ-Y
mit
X = R₂N, RNH oder NH₂
Y = R₂N oder RNH oder NH₂
R = unabhängig voneinander ein verzweigter, unverzweigter oder ringförmiger Alkyl-, Alkenyl-, Alkinyl- oder aromatischer Substituent ist, wobei dieser auch ein oder mehrere Heteroatome enthalten kann;
n= 0 bis 20, vorzugsweise 0 bis 18;und/oder
h.wobei gemäß c. der Anteil der nukleinsäurebindenen Liganden im Verhältnis zu den Verdünnungsliganden ≤ 25%, ≤ 15%, ≤ 10% oder nur ≤ 5% beträgt oder gemäß d. ≤ 50%, ≤ 25%, ≤ 15%, ≤ 10%.

14. Verwendung nach Anspruch 12 oder 13, **gekennzeichnet durch** ein oder mehrere der nachfolgenden Merkmale:
a) die Isolierung und/oder Aufreinigung einer definierten Menge an Nukleinsäuren aus einer nukleinsäurehaltigen Probe wird im Anschluss an ein Nukleinsäureaufreinigungsverfahren durchgeführt, um bereits aufgereinigte Nukleinsäuren zu normalisieren und so eine definierte Menge an Nukleinsäuren zu erhalten;
b) die normalisierte Nukleinsäure wird für eine enzymatische Reaktion wie einer Nukleinsäureamplifikation, Modifikationsreaktion oder Sequenzierreaktionen eingesetzt;
c) bei der Nukleinsäure handelt es sich um PCR-Fragmente.

15. Verwendung eines Kits zur Isolierung und/oder Aufreinigung einer definierten Menge an Nukleinsäuren aus einer nukleinsäurehaltigen Probe in einem Verfahren nach einem oder mehreren der Ansprüche 1-11, wobei das Kit eine nukleinsäurebindende Phase wie in Anspruch 12 oder 13 definiert aufweist, wobei es sich bei der nukleinsäurebindenden Phase um mit N-Propyl-1,3-Propandiaminen modifizierte Polymerpartikel handelt.

16. Verwendung nach Anspruch 15, **dadurch gekennzeichnet, dass** das Kit wenigstens eines der folgenden Merkmale aufweist:
a. einen Bindungspuffer oder eine Bindelösung mit einem pH-Wert, der wenigstens eine pH Einheit unterhalb des pKs-Wertes wenigstens einer der protonierbaren Gruppen der nukleinsäurebindenden Phase liegt, und/oder einen Bindungspuffer oder eine Bindungslösung, der die Einstellung eines solchen pH-Wertes in der Probe ermöglicht; und/oder
b. einen Elutionspuffer oder eine Elutionslösung mit einem pH-Wert, der eine pH Einheit unterhalb des pKs-Wertes wenigstens einer der protonierbaren Gruppen der nukleinsäurebindenden Phase liegt, und/oder einen Elutionspuffer oder eine Elutionslösung, der die Einstellung eines solchen pH-Wertes in der Probe ermöglicht.

17. Verwendung nach Anspruch 15 oder 16, **dadurch gekennzeichnet, dass**
a. der Bindungspuffer oder die Bindungslösung wenigstens eines der folgenden Merkmale aufweist:
i. einen pH-Wert von 3 bis 8; und/oder
ii. einen pH-Wert von 4 bis 7,5; und/oder
iii. einen pH-Wert von 4,5 bis 7; und/oder
iv. einen pH-Wert von 5,5 bis 7; und/oder
v. einen pH-Wert von 6,5 bis 7; und/oder
vi. eine Salzkonzentration von ≤ 1M, ≤ 0,5M, ≤ 0,25M oder ≤ 0,1 M;
und/oder
b. der Elutionspuffer oder die Elutionslösung wenigstens eines der folgenden Merkmale aufweist:
i. einen pH-Wert von 7,5 bis 10; und/oder
ii. einen pH-Wert von 8 bis 9; und/oder
iii. einen pH-Wert von 8,2 bis 8,8; und/oder
iv. eine Salzkonzentration von ≤ 1M, ≤ 0,5M, ≤ 0,25M, ≤ 0,1M, ≤ 25mM, ≤ 15mM oder ≤ 10mM; und/oder
v. er ist ausgewählt aus der Gruppe bestehend aus Wasser, biologischem und organischem Puffer.

## Claims

1. Method for the isolation and/or purification of a defined quantity of nucleic acids from a nucleic acid containing sample, comprising at least the following steps:
a. Contacting the nucleic acid containing sample with a defined quantity of a nucleic acid binding phase with the following characteristics:
(i) the nucleic acid binding phase comprises nucleic acid binding ligands that comprise at least one protonatable group;
(ii) the nucleic acid binding ligands are present on a support in a bound form;
(iii) the nucleic acid binding phase comprises a surface with a low charge density, wherein the low charge density is achieved with one or more of the following characteristics:
aa) the nucleic acid binding ligands, bound to the support, each comprise no more than one or two protonatable groups for binding the nucleic acids; and/or
bb) the nucleic acid binding ligands are selected from the group of mono- and diamines; and/or
cc) the support is coated with a mixture of nucleic acid binding ligands and dilution ligands, wherein the dilution ligands do not bind nucleic acids or bind the nucleic acids with a lower affinity than the nucleic acid binding ligands, and wherein the proportion of nucleic acid binding ligands compared to the dilution ligands is ≤ 50%,
wherein the quantity of nucleic acids in the sample exceeds the binding capacity of the quantity of the nucleic acid binding phase used;
b. binding the nucleic acids to the nucleic acid binding phase at a pH value (binding pH value) that lies below the pKa value of at least one of the protonatable groups;
c. elution of the nucleic acids at a pH value that lies above the binding pH value, wherein a defined quantity of nucleic acids is obtained.

2. The method according to claim 1, **characterised in that** according to cc) the proportion of nucleic acid binding ligands in comparison with the dilution ligands is ≤ 25%, ≤ 15%, ≤ 10% or just ≤ 5%.

3. The method according to claim 1 or 2, **characterised in that** the protonatable group(s) comprise one or more of the following characteristics:
a. a pKa value of 9 to 12;
b. a pKa value of 10 to 12;
c. the protonatable groups are amino groups and are not present in conjunction with the groups that reduce electron density.

4. The method according to one or more of the claims 1 to 3, **characterised in that** the nucleic acid binding phase comprises at least one or more of the following characteristics:
a. the support and/or the nucleic acid binding ligands comprise functional groups that support the release of nucleic acids at the elution pH value, namely cation exchangers as functional groups, in particular acidic groups such as preferably carboxyl groups;
b. the nucleic acid binding ligands are selected from the group consisting of primary, secondary and tertiary amines with the formulas
R₃N, R₂NH, RNH₂ and/or X-(CH₂)ₙ-Y
with
X=R₂N, RNH or NH₂
Y = R₂N or RNH or NH₂
R = independently from each other a branched, unbranched or cyclic alkyl, alkenyl, alkinyl or aromatic substituent, wherein the same can also comprise one or more heteroatoms;
n= 0 to 20, preferably 0 to 18;
c. the support has no own nucleic acid binding features apart from the nucleic acid binding ligands;
d. the support is selected from the group consisting of organic polymers such as polystyrol and its derivatives, polyacrylates and methacrylates and their derivatives, polyurethanes, nylon, polyethylene, polypropylene, polybutylene and copolymers of these materials, polysaccharides and hydrogels such as agarose, cellulose, dextran, sephadex, sephacryl, chitosan, anorganic supports, glass, metal- and metalloid-oxides, in particular Al₂O₃, TiO₂, silica, boron oxide, supports with metal surfaces such as for example gold and magnetic particles and mixtures thereof, tubules, membranes, fleeces, paper, reaction vessels, multiplates, chips and microarrays; and/or
e. the binding capacity lies within a range of 0.1 to 500 µg of nucleic acid per mg of nucleic acid binding phase, preferably 0.1 to 10 µg of nucleic acid per mg of nucleic acid binding phase; and/or
f. the binding capacity lies within a range of 0.01 to 10µg, 0.025 to 10µg, 0.05 to 10µg or 0.1 to 10µg of nucleic acid per mg of nucleic acid binding phase.

5. The method according to one or more of the claims 1 to 4, **characterised in that** binding occurs under conditions that comprise at least one of the following characteristics:
a. binding occurs at a pH value of 3 to 8; and/or
b. binding occurs at a pH value of 4 to 7.5; and/or
c. binding occurs at a pH value of 4.5 to 7; and/or
d. binding occurs at a pH value of 5.5 to 7; and/or
e. binding occurs at a pH value of 6.5 to 7; and/or
f. binding occurs at a salt concentration of ≤ 1M, ≤ 0.5M, ≤ 0.25M or ≤ 0.1 M.

6. The method according to one or more of the claims 1 to 5, **characterised in that** elution takes place under conditions that comprise at least one of the following characteristics:
a. elution takes place at a pH value that lies above the binding pH value, but at least one pH unit below the pKa value of at least one of the protonatable groups; and/or
b. elution takes place at a pH value of 7.5 to 10; and/or
c. elution takes place at a pH value of 8 to 9; and/or
d. elution takes place at a pH value of 8.2 to 8.8; and/or
e. elution takes place at a salt concentration of ≤ 1M, ≤ 0.5M, ≤ 0.25M, ≤ 0.1M, ≤ 25mM, ≤ 15mM or ≤ 10mM; and/or
f. a solution selected from the group of water, biological and organic buffer is used for elution.

7. The method according to one or more of the claims 1 to 6, **characterised in that** the method is carried out following a nucleic acid purification method in order to normalise nucleic acids already purified and to thereby obtain a defined quantity of nucleic acids.

8. The method according to one or more of the claims 1 to 7, **characterised in that** the normalised nucleic acid is used for an enzymatic reaction, preferably in a nucleic acid amplification, modification reaction or sequencing reaction.

9. The method according to one or more of the claims 1 to 8, **characterised in that** the nucleic acid consists of PCR fragments.

10. The method according to one or more of the preceding claims, **characterised in that** the support is provided by non-magnetic or magnetic particles.

11. The method according to one or more or the preceding claims, **characterised in that** the nucleic acid binding ligand is N-propyl-1,3 propandiamine.

12. Use of a nucleic acid binding phase for the isolation and/or purification of a defined quantity of nucleic acids from a nucleic acid containing sample, **characterised in that** the nucleic acid binding phase comprises nucleic acid binding ligands with at least one protonatable group, wherein the nucleic acid binding ligands are present bound to a support and the nucleic acid binding phase comprises a surface with a low charge density, wherein the low charge density is achieved with one or more of the following characteristics:
a. the nucleic acid binding ligands, bound to the support, each comprise no more than one or two protonatable groups for binding the nucleic acids; and/or
b. the nucleic acid binding ligands are selected from the group of mono- and diamines; and/or
c. the support is coated with a mixture of nucleic acid binding ligands and dilution ligands, wherein the dilution ligands do not bind nucleic acids or bind the nucleic acids with a lower affinity than the nucleic acid binding ligands, and wherein the proportion of nucleic acid binding ligands in comparison with the dilution ligands is ≤ 50%;
wherein an elution pH value that lies above the binding pH value is set, and wherein the quantity of nucleic acid binding phase is selected in such a way that the nucleic acids in the sample are present in excess of the binding capacity of the nucleic acid binding phase used.

13. Use according to claim 12, **characterised in that** the nucleic acid binding phase comprises at least one of the following characteristics:
a. the protonatable group(s) has/have a pKa value of 9 to 12, preferably a pKa value of 10 to 12; and/or
b. the protonatable groups are amino groups and are not present in conjugation with groups reducing the electron density; and/or
c. the nucleic acid binding phase comprises functional groups that favour the release/elution of the nucleic acids at the elution pH value, preferably cation exchangers, in particular carboxyl groups; and/or
d. the binding capacity of the nucleic acid binding phase lies within a range of 0.1 to 500 µg nucleic acid per mg of nucleic acid binding phase, preferably 0.1 to 200 µg, more preferably 0.1 to 10 µg of nucleic acid per mg of nucleic acid binding phase;
e. the support has no own nucleic acid binding features apart from the nucleic acid binding ligands; and/or
f. the support is selected from the group consisting of organic polymers such as polystyrol and its derivatives, polyacrylates and methacrylates and their derivatives, polyurethanes, nylon, polyethylene, polypropylene, polybutylene and copolymers of these materials, polysaccharides and hydrogels such as agarose, cellulose, dextran, sephadex, sephacryl, chitosan, anorganic supports, glass or other metal- and metalloid-oxides, in particular Al₂O₃, TiO₂, silica, boron oxide, supports with metal surfaces such as for example gold and magnetic particles or mixtures thereof, tubules, membranes, fleeces, paper, reaction vessels, multiplates, chips and microarrays; and/or
g. the nucleic acid binding ligands are selected from the group consisting of primary, secondary and tertiary amines with the formulas
R₃N, R₂NH, RNH₂ and/or X-(CH₂)ₙ-Y
with
X = R₂N, RNH or NH₂
Y = R₂N or RNH or NH₂
R = independently from each other a branched, unbranched or cyclic alkyl, alkenyl, alkinyl or aromatic substituent, wherein the same can also comprise one or more heteroatoms;
n= 0 to 20, preferably 0 to 18; and/or
h. wherein, according to c., the proportion of nucleic acid binding ligands in comparison to the dilution ligands is ≤ 25%, ≤ 15%, ≤ 10% or just ≤ 5%, or, according to d., ≤ 50%, ≤ 25%, ≤ 15%, ≤ 10%.

14. Use according to claim 12 or 13, **characterised by** one or more of the following characteristics:
a) the isolation and/or purification of a defined quantity of nucleic acids from a nucleic acid containing sample is carried out following a nucleic acid purification method in order to normalise already purified nucleic acids to thereby obtain a defined quantity of nucleic acids;
b) the normalised nucleic acid is used for an enzymatic reaction such as a nucleic acid amplification, modification reaction or sequencing reaction;
c) the nucleic acid consists of PCR fragments.

15. Use of a kit for the isolation and/or purification of a defined quantity of nucleic acids from a nucleic acid containing sample in a method according to one or more of the claims 1-11, wherein the kit comprises a nucleic acid binding phase as defined in claims 12 or 13, wherein the nucleic acid binding phase consists of polymer particles modified with N-propyl-1,3-propandiamine.

16. Use according to claim 15, **characterised in that** the kit comprises at least one of the following characteristics:
a. a binding buffer or a binding solution with a pH value that lies at least one pH unit below the pKa value of at least one of the protonatable groups of the nucleic acid binding phase and/or a binding buffer or a binding solution that enables the setting of such a pH value in the sample; and/or
b. an elution buffer or an elution solution with a pH value that lies one pH unit below the pKa value of at least one of the protonatable groups of the nucleic acid binding phase and/or an elution buffer or an elution solution that enables the setting of such a pH value in the sample.

17. Use according to claim 15 or 16, **characterised in that**
a. the binding buffer or the binding solution comprises at least one of the following characteristics:
i. a pH value of 3 to 8; and/or
ii. a pH value of 4 to 7.5; and/or
iii. a pH value of 4.5 to 7; and/or
iv. a pH value of 5.5 to 7; and/or
v. a pH value of 6.5 to 7; and/or
vi. a salt concentration of ≤ 1M, ≤ 0.5M, ≤ 0.25M or ≤ 0.1 M;
and/or
b. the elution buffer or the elution solution comprise at least one of the following characteristics:
i. a pH value of 7.5 to 10; and/or
ii. a pH value of 8 to 9; and/or
iii. a pH value of 8.2 to 8.8; and/or
iv. a salt concentration of ≤ 1M, ≤ 0.5M, ≤ 0.25M, ≤ 0.1M, ≤ 25mM, ≤15mM or ≤ 10mM; and/or
v. it is selected from the group consisting of water, biological and organic buffer.

## Revendications

1. Procédé d'isolement et/ou de purification d'une quantité définie d'acides nucléiques d'un échantillon contenant des acides nucléiques, qui présente au minimum les étapes suivantes :
a. La mise en contact de l'échantillon contenant des acides nucléiques avec une quantité définie d'une phase liant les acides nucléiques avec les caractéristiques suivantes :
(i) la phase liant les acides nucléiques présente des ligands liant les acides nucléiques, qui comportent au moins un groupe protonable ;
(ii) les ligands liant les acides nucléiques sont liés sur un support ;
(iii) la phase liant les acides nucléiques présente une surface avec une faible densité de charge, la faible densité de charge étant obtenue par une ou plusieurs des caractéristiques suivantes :
aa) les ligands liant les acides nucléiques comportent chacun, liés au support, pas plus qu'un ou deux groupes protonables pour la liaison des acides nucléiques ; et/ou
bb) les ligands liant les acides nucléiques sont choisis parmi le groupe des mono- et diamines ; et/ou
cc) le support est revêtu d'un mélange de ligands liant les acides nucléiques et de ligands de dilution, les ligands de dilution ne liant pas les acides nucléiques ou liant les acides nucléiques avec une affinité plus faible que les ligands liant les acides nucléiques, et la fraction de ligands liant les acides nucléiques étant ≤ 50 % par rapport aux ligands de dilution,
où la quantité d'acides nucléiques dans l'échantillon dépasse la capacité de liaison de la quantité utilisée de phase liant les acides nucléiques ;
b. La liaison des acides nucléiques à la phase liant les acides nucléiques à un pH (pH de liaison), qui est inférieur au pKa d'au moins un des groupes protonables ;
c. L'élution des acides nucléiques à un pH, qui est supérieur au pH de liaison, obtenant une quantité définie d'acides nucléiques.

2. Procédé selon la revendication 1, **caractérisé en ce que,** conformément à cc) la fraction de ligands liant les acides nucléiques est ≤ 25 %, ≤ 15 %, ≤ 10 % ou seulement ≤ 5% par rapport aux ligands de dilution.

3. Procédé selon la revendication 1 ou 2, **caractérisé en ce que** le(s) groupe(s) protonable(s) présente(nt) une ou plusieurs des caractéristiques suivantes :
a. un pKa de 9 à 12 ;
b. un pKa de 10 à 12 ;
c. les groupes protonables sont des groupes amino et ne sont pas en conjugaison avec les groupes diminuant la densité électronique.

4. Procédé selon une ou plusieurs des revendications 1 à 3, **caractérisé en ce que** la phase liant les acides nucléiques présente au moins une ou plusieurs des caractéristiques suivantes :
a. le support et/ou les ligands liant les acides nucléiques comportent des groupes fonctionnels, qui favorisent la libération des acides nucléiques au pH d'élution, à savoir des échangeurs de cations comme groupes fonctionnels, en particulier des groupes acides tels que de préférence des groupes carboxyle ;
b. les ligands liant les acides nucléiques sont choisis parmi le groupe constitué par des amines primaires, secondaires et tertiaires de formules
R₃N, R₂NH, RNH₂ et/ou X-(CH₂)ₙ-Y
avec
X = R₂N, RNH ou NH₂
Y = R₂N ou RNH ou NH₂
R = indépendamment l'un de l'autre un substituant alkyle, alcényle, alcynyle ou aromatique ramifié, non ramifié ou cyclique, celui-ci pouvant contenir un ou plusieurs hétéroatomes ;
n = 0 à 20, de préférence 0 à 18 ;
c. le support ne présente pas de propriétés intrinsèques de liaison d'acides nucléiques en plus des ligands liant les acides nucléiques ;
d. le support est choisi parmi le groupe constitué par des polymères organiques comme le polystyrène et ses dérivés, les polyacrylates et -méthacrylates et leurs dérivés, les polyuréthanes, le nylon, le polyéthylène, le polypropylène, le polybutylène et les copolymères de ces matières, des polysaccharides et des hydrogels comme l'agarose, la cellulose, le dextran, le Sephadex, le Sephacryl, le chitosan, des supports inorganiques, de verre, des oxydes métalliques et semi-métalliques, en particulier Al₂O₃, TiO₂, silice, oxyde de bore, des supports avec surfaces métalliques, comme par exemple, l'or et les particules magnétiques et des mélanges de ceux-ci, des tubes, des membranes, des non-tissés, de papier, des réacteurs, Multiplates, des puces et des microréseaux ; et/ou
e. la capacité de liaison est comprise dans un intervalle de 0,1 à 500 µg d'acides nucléiques par mg de phase liant les acides nucléiques, de manière particulièrement préférée 0,1 à 10 µg d'acides nucléiques par mg de phase liant les acides nucléiques ; et/ou
f. la capacité de liaison est comprise dans un intervalle de 0,01 à 10 µg, de 0,025 à 10 µg, de 0,05 à 10 μg ou de 0,1 à 10 μg d'acides nucléiques par mg de phase liant les acides nucléiques

5. Procédé selon une ou plusieurs des revendications 1 à 4, **caractérisé en ce que** la liaison s'effectue dans des conditions qui présentent au moins une des caractéristiques suivantes :
a. la liaison s'effectue à un pH de 3 à 8 ; et/ou
b. la liaison s'effectue à un pH de 4 à 7,5 ; et/ou
c. la liaison s'effectue à un pH de 4,5 à 7 ; et/ou
d. la liaison s'effectue à un pH de 5,5 à 7 ; et/ou
e. la liaison s'effectue à un pH de 6,5 à 7 ; et/ou
f. la liaison s'effectue à une concentration en sel de ≤ 1 M, de ≤ 0,5 M, de ≤ 0,25 M ou de ≤ 0,1 M.

6. Procédé selon une ou plusieurs des revendications 1 à 5, **caractérisé en ce que** l'élution s'effectue dans des conditions qui présentent au moins une des caractéristiques suivantes :
a. l'élution s'effectue à un pH qui est supérieur au pH de liaison, cependant inférieur d'au moins une unité de pH au pKa d'au moins un des groupes protonables ; et/ou
b. l'élution s'effectue à un pH de 7,5 à 10 ; et/ou
c. l'élution s'effectue à un pH de 8 à 9 ; et/ou
d. l'élution s'effectue à un pH de 8,2 à 8,8 ; et/ou
e. l'élution s'effectue à une concentration en sel de ≤ 1 M, de ≤ 0,5 M, de ≤ 0,25 M, de ≤ 0,1 M, de ≤ 25 mM, de ≤ 15 mM ou de ≤ 10 mM ; et/ou
f. une solution est utilisée pour l'élution, qui est choisie parmi le groupe constitué par l'eau, un tampon biologique et organique.

7. Procédé selon une ou plusieurs des revendications 1 à 6, **caractérisé en ce que** le procédé est effectué à la suite d'un procédé de purification d'acides nucléiques pour normaliser les acides nucléiques déjà purifiés et obtenir ainsi une quantité définie d'acides nucléiques.

8. Procédé selon une ou plusieurs des revendications 1 à 7, **caractérisé en ce que** l'acide nucléique normalisé est utilisé pour une réaction enzymatique, de préférence dans une amplification d'acides nucléiques, une réaction de modification ou une réaction de séquençage.

9. Procédé selon une ou plusieurs des revendications 1 à 8, **caractérisé en ce que** l'acide nucléique est un fragment de PCR.

10. Procédé selon une ou plusieurs des revendications précédentes, **caractérisé en ce que** le support est préparé par des particules non magnétiques ou magnétiques.

11. Procédé selon une ou plusieurs des revendications précédentes, **caractérisé en ce que** le ligand liant les acides nucléiques est la N-propyl-1,3 propanediamine.

12. Utilisation d'une phase liant les acides nucléiques pour l'isolement et/ou la purification d'une quantité définie d'acides nucléiques d'un échantillon contenant des acides nucléiques, **caractérisée en ce que** la phase liant les acides nucléiques présente des ligands liant les acides nucléiques avec au moins un groupe protonable, où les ligands liant les acides nucléiques sont liés sur un support et la phase liant les acides nucléiques présente une surface avec une faible densité de charge, la faible densité de charge étant obtenue par une ou plusieurs des caractéristiques suivantes :
a. les ligands liant les acides nucléiques comportent chacun, liés au support, pas plus qu'un ou deux groupes protonables pour la liaison des acides nucléiques ; et/ou
b. les ligands liant les acides nucléiques sont choisis parmi le groupe des mono- et diamines ; et/ou
c. le support est revêtu d'un mélange de ligands liant les acides nucléiques et de ligands de dilution, les ligands de dilution ne liant pas les acides nucléiques ou liant les acides nucléiques avec une affinité plus faible que les ligands liant les acides nucléiques, et la fraction de ligands liant les acides nucléiques étant ≤ 50 % par rapport aux ligands de dilution ;
où un pH d'élution est ajusté, qui est supérieur au pH de liaison et où la quantité de phase liant les acides nucléiques est choisie de manière à ce que les acides nucléiques dans l'échantillon soient en excès par rapport à la capacité de liaison de la phase liant les acides nucléiques utilisée.

13. Utilisation selon la revendication 12, **caractérisée en ce que** la phase liant les acides nucléiques présente au moins une des caractéristiques suivantes ;
a. le(s) groupe(s) protonable(s) présente(nt) un pKa de 9 à 12, de préférence un pKa de 10 à 12 ; et/ou
b. les groupes protonables sont des groupes amino et ne sont pas en conjugaison avec les groupes diminuant la densité électronique ; et/ou
c. la phase liant les acides nucléiques présente des groupes fonctionnels qui favorisent au pH d'élution la libération/l'élution des acides nucléiques, de préférence des échangeurs de cations, en particulier des groupes carboxyle ; et/ou
d. la capacité de liaison de la phase liant les acides nucléiques est comprise dans un intervalle de 0,1 à 500 μg d'acide nucléique par mg de phase liant les acides nucléiques, de préférence de 0,1 à 200 μg, de manière particulièrement préférée de 0,1 à 10 μg d'acide nucléique par mg de phase liant les acide nucléiques ;
e. le support ne présente pas de propriétés intrinsèques de liaison d'acides nucléiques en plus des ligands liant les acides nucléiques ; et/ou
f. le support est choisi parmi le groupe constitué par des polymères organiques comme le polystyrène et ses dérivés, les polyacrylates et -méthacrylates et leurs dérivés, les polyuréthanes, le nylon, le polyéthylène, le polypropylène, le polybutylène et les copolymères de ces matières, des polysaccharides et des hydrogels comme l'agarose, la cellulose, le dextran, le Sephadex, le Sephacryl, le chitosan, des supports inorganiques, de verre ou autres oxydes métalliques et semi-métalliques, en particulier Al₂O₃, TiO₂, silice, oxyde de bore, des supports avec surfaces métalliques, comme par exemple l'or, les particules magnétiques ou des mélanges de ceux-ci, des tubes, des membranes, des non-tissés, de papier, des réacteurs, Multiplates, des puces et des microréseaux ; et/ou
g. les ligands liant les acides nucléiques sont choisis parmi le groupe constitué d'amines primaires, secondaires et tertiaires de formules
R₃N, R₂NH, RNH₂ et/ou X-(CH₂)ₙ-Y
avec
X = R₂N, RNH ou NH₂
Y = R₂N ou RNH ou NH₂
R = indépendamment l'un de l'autre un substituant alkyle, alcényle, alcynyle ou aromatique ramifié, non ramifié ou cyclique, celui-ci pouvant contenir un ou plusieurs hétéroatomes ;
n= 0 à 20, de préférence 0 à 18 ; et/ou
h. où selon c. la fraction de ligands liant les acides nucléiques est ≤ 25 %, ≤ 15 %, ≤ 10 % ou seulement ≤ 5 % par rapport aux ligands de dilution ou selon d. ≤ 50 %, ≤ 25 %, ≤ 15 %, ≤ 10 %.

14. Utilisation selon la revendication 12 ou 13, **caractérisée par** une ou plusieurs des caractéristiques suivantes :
a) l'isolement et/ou la purification d'une quantité définie d'acides nucléiques d'un échantillon contenant des acides nucléiques est effectuée à la suite d'un procédé de purification d'acides nucléiques, pour normaliser les acides nucléiques déjà purifiés et obtenir ainsi une quantité définie d'acides nucléiques ;
b) l'acide nucléique normalisé est utilisé pour une réaction enzymatique telle qu'une amplification d'acides nucléiques, une réaction de modification ou une réaction de séquençage ;
c) quant à l'acide nucléique il s'agit de fragments de PCR.

15. Utilisation d'une trousse pour l'isolement et/ou la purification d'une quantité définie d'acides nucléiques d'un échantillon contenant des acides nucléiques dans un procédé selon une ou plusieurs des revendications 1 à 11, où la trousse présente une phase liant les acides nucléiques comme définie dans la revendication 12 ou 13, la phase liant les acides nucléiques étant des particules de polymère modifiées avec des N-propyl-1,3-propanediamines.

16. Utilisation selon la revendication 15, **caractérisée en ce que** la trousse présente au moins une des caractéristiques suivantes :
a. un tampon de liaison ou une solution de liaison avec un pH qui est inférieur d'au moins une unité de pH au pKa d'au moins un des groupes protonables de la phase liant les acides nucléiques, et/ou un tampon de liaison ou une solution de liaison, qui permet l'ajustement d'un tel pH dans l'échantillon ; et/ou
b. un tampon d'élution ou une solution d'élution avec un pH qui est inférieur d'une unité de pH au pKa d'au moins une des groupes protonables de la phase liant les acides nucléiques, et/ou un tampon d'élution ou une solution d'élution, qui permet l'ajustement d'un tel pH dans l'échantillon.

17. Utilisation selon la revendication 15 ou 16, **caractérisée en ce que**
a. le tampon de liaison ou la solution de liaison présente au moins une des caractéristiques suivantes ;
i. un pH de 3 à 8 ; et/ou
ii. un pH de 4 à 7,5 ; et/ou
iii. un pH de 4,5 à 7 ; et/ou
iv. un pH de 5,5 à 7 ; et/ou
v. un pH de 6,5 à 7 ; et/ou
vi. une concentration en sel de ≤ 1 M, de ≤ 0,5 M, de ≤ 0,25 M ou de ≤ 0,1 M ;
et/ou
b. le tampon d'élution ou la solution d'élution présente au moins une des caractéristiques suivantes :
i. un pH de 7,5 à 10 ; et/ou
ii. un pH de 8 à 9 ; et/ou
iii. un pH de 8,2 à 8,8 ; et/ou
iv. une concentration en sel de ≤ 1 M, de ≤ 0,5 M, de ≤ 0,25 M, de ≤ 0,1 M, de ≤ 25 mM, de ≤ 15 mM ou de ≤ 10 mM ; et/ou
v. il est choisi parmi le groupe constitué par l'eau, un tampon biologique et organique.
